Europäisches Patentamt

European Patent Office

Office européen des brevets

(11) Publication number: **0 051 496**
**B1**

(12) **EUROPEAN PATENT SPECIFICATION**

(45) Date of publication of patent specification: **05.02.86**

(51) Int. Cl.⁴: **G 01 N 35/02**, G 01 N 33/53, G 01 N 33/80

(21) Application number: **81305238.8**

(22) Date of filing: **04.11.81**

(54) Analyzing method and apparatus for immunological agglutinating reaction.

(30) Priority: **04.11.80 JP 155021/80**
**29.12.80 JP 187551/80**
**29.12.80 JP 187552/80**

(43) Date of publication of application:
**12.05.82 Bulletin 82/19**

(45) Publication of the grant of the patent:
**05.02.86 Bulletin 86/06**

(84) Designated Contracting States:
**DE FR GB**

(56) References cited:
**EP-A-0 050 018**
**CH-A- 492 214**
**DE-A-3 022 940**
**FR-A-2 423 769**
**US-A-3 351 177**
**US-A-3 533 744**
**US-A-3 617 222**
**US-A-3 909 201**
**US-A-4 040 533**

(73) Proprietor: **OLYMPUS OPTICAL CO., LTD.**
**43-2, 2-chome, Hatagaya Shibuya-ku**
**Tokyo 151 (JP)**

(72) Inventor: **Kano, Tokio**
**No. 2388-2, Haijima-Cho**
**Akishima City Tokyo (JP)**
Inventor: **Tamagawa, Akira Hirayama-House**
**224**
**No. 7-7 Higashihinayama 1-Chome**
**Hino City Tokyo (JP)**

(74) Representative: **Fane, Christopher Robin King et al**
**HASELTINE LAKE & CO. Hazlitt House 28**
**Southampton Buildings Chancery Lane**
**London, WC2A 1AT (GB)**

Courier Press, Leamington Spa, England.

# Description

This invention relates to agglutination analyzing method and apparatus for analyzing agglutination patterns produced in response to immunological agglutinating reaction and more particularly to method and apparatus for identifying various kinds of blood types with the aid of agglutination patterns of blood corpuscles or for detecting various kinds of antibodies and various antigens in sample solutions (like viruses, proteins or the like) with the aid of agglutination patterns of not only blood corpuscles but also of particles of materials such as latex, carbon or the like.

In known analyzing methods for the agglutinating reaction, reaction vessels and analyzing steps are different for respective components to be analyzed. For instance, in a known manual method for judging the blood type of ABO system, use was made of test tubes as the reaction vessels. In this method a sample blood is first centrifuged to separate red blood cells and serum from each other, and then a given amount of blood cells is mixed with a diluent to form a blood cell suspension of 2 to 5%. Then, a given amount of the blood cell suspension is delivered into a test tube into which A-type antiserum or B-type antiserum is also distributed. After the blood cells have been centrifuged, the test tube is shaken and it is confirmed with naked eyes whether or not agglutination is formed. In this case, the sample blood which produces agglutination together with A-type antibody, but does not produce agglutination together with B-type antibody is identified as A-Type, the sample blood which produces agglutination exclusively with B-type antibody is judged to be B-type, the sample blood which forms agglutination with both A-type and B-type antibodies is determined to be AB-type, and sample blood which does not produce agglutination with either A-type or B-type antibodies is judged as O-type.

In order to detect and measure HBs antigen, a method has been proposed which makes use of a plate, called a microplate, made of plastics and provided with a number of wells, i.e. reaction vessels each having a conical base surface. This conventional method makes use of a microplate having 10×12 wells, for example, and detects and determines the HBs antigen by the following procedure.

1) Buffer solution specially prescribed for R-PHA method is introduced into each well of the microplate one drop (0.025 ml) at a time.

2) A test serum (0.025 ml) is added to the first well of a row. By using a diluter, the doubling dilution is performed along the row up to the last (tenth) well.

3) One drop of R-PHA buffer (0.025 ml) is added to a first row and one drop of R-PHA inhibition solution is added to a second row.

(4) After the mixtures thus treated have been sufficiently agitated by a micromixer for 10 seconds, incubation is effected for one hour at 37°C.

(5) A drop of R-PHA cells of 1% suspension (0.025 ml) is added to each well.

(6) The mixtures are agitated by the micromixer for ten seconds to suspend the R-PHA cells uniformly.

(7) After the mixtures thus treated have remained stationary at room temperature for one hour, agglutination patterns are detected.

In the T-PHA system for syphilis, different diluents of a sample serum are formed in the microplate and a reagent prepared by bonding syphilus virus with red blood cells of sheep is added to the serum diluents. After natural segmentation, it is confirmed with naked eyes whether or not agglutination is formed.

French Patent Specification 2423769 discloses a microplate for agglutination reaction and including a plurality of receptacles in transparent plastics and with a substantially cylindrical side wall and a concave bottom. The concave bottom includes a central part in the form of part of a sphere merging without angular discontinuity into a frusto-conical part the angle of inclination of which is substantially equal to the limit angle of normal agglutination. This microplate is not suitable for analysis by agglutination patterns on an advancing conveyor, since even slight shocks can disturb the patterns by causing agglutinated particles to move down the smooth surface of the concave bottom.

As described above, in the analysing methods for immunological agglutination reaction different kinds of reaction vessels are used depending upon the test items and further successive steps are also different for respective items.

There has also been known a microtiter method in which use is made of the microplate as the reaction vessels and steps are partially automated. In this method, delivery of samples and reagents and detection of agglutination are carried out automatically, but other steps are effected manually. This is due to the fact that in case of using the microplate, mechanisms and operations are liable to be complicated and thus, it is extremely difficult to effect all the steps automatically. Further, the microtiter method has several disadvantages that since the sample serum is delivered quantitatively with the aid of capillary phenomenon, it is necessary to deliver previously diluent into each well in the microplate and then a tip of dilutor onto which a sample has been applied is immersed into the diluent to mix the serum and diluent. Such a step is very complicated as compared with normal delivery steps in the analyzing apparatuses and thus could be controlled only by means of complicated mechanisms. Further, the delivery amount is made always constant, because the capillarity is utilized and thus, the delivery amount could not be adjusted at will. Further, the mixed solution is applied to the dilutor and the sample is partially wasted. This becomes a serious drawback in a multi-item analyzer.

Moreover, if the delivery of the blood cell sample is effected before the serum sample delivery, indefinite amount of the blood cell sample might be removed from the well. Therefore, in the microtiter system, the diluent delivery, serum delivery and blood cell delivery have to be performed in this order and thus, the mechanical arrangement or design might be restricted. Further in the microtiter method, since use is made of the blood cell suspension of about 1%, the operation is liable to be very complicated as compared with the test tube method described above.

In a conventional method of identifying blood types, for example, which has heretofore been proposed, use was made of a winecup-shaped reaction vessel into which was quantitatively introduced a sample solution, i.e. 2 to 5% of test blood corpuscles suspended in saline solution, and a specified antiserum, i.e. A-type or B-type antiserum. Then, the mixture remained stationary for reaction between blood corpuscles and antiserum. Subsequently, it was centrifuged to sediment blood corpuscles. Then, the reaction vessel was rapidly wobbled such that the sedimented blood corpuscles were forcedly separated one from the other and then relatively slowly wobbled so as to collect the clumped compositions in the center portion of the base surface of the vessel and form settling patterns, thereby enabling photometric detection of these patterns.

Such conventional blood type identifying method in which sedimentation is effected and then the reaction vessel is rapidly wobbled so as to separate the sedimented blood corpuscles from the base surface of the vessel can only be applied to the analysis of regular ABO blood type which shows strong agglutination, but could not be applied to many other immunological agglutination reactions which show weak agglutination, for example, a method of determining Rh blood subtype or detecting various kinds of incomplete antibodies. That is, if the agglutination reaction is weak, the blood corpuscles or the like which have been clumped together become separated one from another when the reaction vessel is wobbled, and as a result, the particles are not collected in the center portion of the reaction vessel.

Further, in this known method, in order to effect the accurate judgement of the blood type, it is necessary to prepare a substantial amount of the sample blood cells and thus, required amounts of standard anti-serums are increased accordingly. Nowadays, very large numbers of test items are to be effected for respective patients and thus, required amounts of the sample blood for respective items must be decreased as much as possible.

United States Patent 3533744 discloses a serological analyzing apparatus, in which the samples are stored in containers in a refrigerator. The sample containers are syringes in which the samples are collected on taking the samples. The cannula of each syringe is provided with a length of flexible tubing and each syringe is held in a cassette for facilitating the handling. The cassette is provided with at least two identification labels carrying e.g. the number of the sample and the name of the patient from which the sample is taken.

From the refrigerator the cassettes are fed successively by means of a feeding mechanism to a dispensing position adjacent a magazine for sample carriers. The sample carriers are in the form of integral rectangular slides, each having thirty elongate juxtaposed shallow receptacles for receiving fractions of one single sample. The sample carriers are preferably of the disposable type and made of a transparent plastics.

The magazine is provided with a mechanism for displacing one sample carrier at a time out of the magazine and positioning the sample carrier on a conveyor. The drive (not shown) of the conveyor provides an intermittent stop and go movement at a predetermined speed of two parallel, endless conveyor chains which are provided with equally spaced driving pins for engaging holes of the sample carrier.

When a sample carrier is horizontally displaced from the magazine it passes below the free end of the tubing attached to a syringe fed to the dispensing position. During the passage the sample is continuously dispensed from the syringe to the sample fraction receptacles of the sample carrier. The dispensing is effected by means of an actuator arranged to displace the plunger of the syringe upwardly.

If the apparatus is to be used for complete blood typing, the blood samples in the syringes must initially be separated into a plasma (or serum) fraction and a blood cell fraction. This separation can be accomplished by centrifuging the samples in the syringes before the syringes are put into the refrigerator. Alternatively, the syringes can be stored in the refrigerator long enough to allow the blood cells to settle by gravity. In any case the syringes will contain blood plasma in the upper portion thereof and blood cells in the lower portion. Hence, the first sample fraction receptacles passing below the tubing receive plasma. When the desired number of receptacles have received plasma, the displacing mechanism actuates an electromagnetic stirrer positioned adjacent the dispensing position. The stirrer then starts to agitate a magnetic member in the syringe to mix the blood cells with remaining plasma so that during the continued dispensing the receptacles will receive a suspension of blood cells.

When a cassette reaches its dispensing position and a sample carrier is displaced towards the conveyor, one of the two identical identification labels is transferred from the cassette to a conveyor band. The labels are secured to the cassette in vertical position along their upper edges, and when the cassette approaches the dispensing position, a cam element folds the label to horizontal position, whereupon this label is secured to the conveyor band adhesively or by means of clips and severed from the cassette by means of a cutter. The other label remains on the cassette which after the sample has been dispensed is fed to a second refrigerator. The conveyor band is connected to or synchronized with the conveyor to transport the first label side by side with the corresponding sample carrier.

After a sample carrier has been positioned on the conveyor, the conveyor transports the sample carrier step by step to and through a reagent adding station wherein the required reagents are added to the sample fractions from reagent containers. Each container is provided with a dispensing conduit passing through a pump element in a peristaltic pump assembly to a socket in a horizontal, preferably transparent plate overlying the conveyor and the sample carriers transported thereby. The position of the socket in the plate depends on the lateral position of the sample fraction receptacle for which the reagent is intended and of the required reaction time: if a long reaction time is required, the socket is positioned near the rear edge of the plate, whereas if a short reaction time is required, the socket is positioned a greater distance from the rear edge of the plate.

After the reagents have been added the sample carriers must often be agitated so that the sample fractions are mixed with the reagents and, moreover, the sample fractions must often be maintained at predetermined temperatures. The agitation is accomplished by rocking the sample carriers about an axis which is transverse to the direction of movement of the conveyor, i.e. parallel to the row of receptacles in the sample carriers. To this end the apparatus is provided with a plurality of rocking devices which are connected together and spaced in the direction of movement of the conveyor. The rocking devices are positioned so that they underlie and support one sample carrier each during the rest periods of the conveyor.

From the reagent adding station the sample carriers are transported in succession to a reading station, which may comprise means for photometrically determining the optical density of the sample fractions and means for photographing each sample carrier together with the corresponding identification label.

The photometrical determination of the optical density is accomplished by means of a plurality of photoelectric cells, one for each sample fraction receptacle, arranged in a row extending transversely of the direction of movement of the conveyor. The row of photoelectric cells is positioned above the path of movement of the sample carriers which are illuminated from below by a light source, preferably a monochromatic one. The sample fractions in the sample carriers transmit light in dependence on their optical densities, that is in dependence on agglutination caused by the reagents, and thus the photoelectric cells are energized in dependence on the optical densities of the sample fractions. The determination may take place while the sample carriers are stationary, each sample fraction receptacle then being illuminated over its entire surface. Alternatively, the determination can take place while the sample carriers are moving at a constant speed with respect to the photoelectric cells and the light source. In this case each sample fraction receptacle is illuminated through a very narrow transverse slit, so that the sample fractions are scanned by a very thin transverse band of light during the movement of the sample carriers.

The result of the determination of the optical density of the sample fractions can be displayed and/or recorded in any suitable way, e.g. by means of one or more multi-channel pen recorders.

Immediately after the determination of the optical density each sample carrier and the corresponding identification label are photographed.

The system uses the optical density of the sample fractions for analysis, rather than the agglutination patterns, and is thus restricted in its applications. Moreover, the continual intermittent advance of the conveyor during the agglutination reaction makes the system unsuitable for analysis using such patterns.

United States Patent Specification 3,909,201 discloses analysis apparatus for carrying out particle agglutination, flocculation or precipitation for liquid samples, in particular blood samples. Supports provided with a plurality of microcontainers, in which reactions between samples and analysis reagents are carried out, are transferred from a sample and reagent dispensing station to a reaction observation station. Each support, having received a predetermined quantity of sample for analysis and of analysis reagents, is rotated about its axis. The microcontainers are of a shape and size similar to the closed ends of microtubes and thus again are of a winecup shape.

The applicant has proposed in EP—A2—0 050 018, published on 21.4.82, a plate for use in an immunological analysis on the basis of agglutination reaction of particles, which has formed therein a number of reaction vessels each having a conical bottom surface. Each vessel has an inclined bottom surface and a plurality of concentric protrusions or depressions are formed in the bottom surface to form a stable basic layer of settled particles, which basic layer can assure the formation of clear and precise particle patterns on the bottom surface.

The applicant has proposed in a Japanese Patent Application Laid-open Publication No. 146,044/80 (published on 4.5.82) a blood type judging method in which not only blood types due to natural antibodies showing strong agglutination, but also blood types due to incomplete antibodies having weak agglutination can be judged very precisely, while necessary amounts of the blood samples can be minimized.

In this method, use is made of reaction vessels having conical bottom surfaces and blood cells in blood samples to be analyzed are delivered into the reaction vessels together with standard antiserum reagents. After sufficiently mixing the samples and reagents, the mixtures remain stationary for a relatively short time such as thirty minutes and then agglutination patterns formed on the bottom surfaces of reaction vessels are detected to identify the blood type. In this method, when the sample blood cells react with the antiserum, blood cells settling down on the inclined bottom surface of reaction vessel are combined with each other and are deposited uniformly on the bottom in the manner of snow, whilst when the blood cells do not react with the antiserum, the settling cells are not agglutinated and roll down along the inclined bottom surface and are collected at the bottom center. Therefore, by photoelectrically detecting the patterns formed by blood cells settled on the bottom surface, it is possible to identify the blood type. According to this method, since the reaction vessels remain stationary during the reaction and detection steps, various kinds of antibodies and antigens such as HBs antigen and syphilis antibody can be effectively detected.

The present invention has for its object to provide a novel and useful method for effecting fully automatically analysis with immunological agglutionating reaction in a precise and efficient manner.

It is another object of the invention to provide an automatic analyzing method which can identify various kinds of blood types with both strong and weak agglutinations and can detect various kinds of substances such as various antigens, antibodies, viruses and specific proteins.

According to one aspect of the present invention, there is provided an analyzing method for agglutinating reaction comprising

supplying carriers successively into an entrance of a reaction line, each carrier having therein a plurality of reaction vessels each having an inclined bottom surface;

delivering given amounts of sample liquids into the reaction vessels of each carrier, while the carrier is fed along the reaction line;

delivering given amounts of reagents into the reaction vessels of each carrier, while the carrier is fed along the reaction line;

feeding each carrier along the reaction line while the reactions take place in test liquids in the reaction vessels, said test liquids being mixtures of samples and reagents and containing particles;

detecting photoelectrically agglutination formed in the reaction vessels to effect analyses in accordance with existence and non-existence of agglutination; and

discharging each carrier from an exit of the reaction line,

characterized in that said feeding comprises feeding each carrier in a steady manner and thus without subjecting said test liquids in its reaction vessels to sharp shocks, said detecting comprises detecting photoelectrically agglutination patterns formed on the bottom surfaces of the reaction vessels by the particles, and the bottom surface of each reaction vessel is inclined to the horizontal in such a manner and is provided with a plurality of such steps that the motion of the agglutinated particles down the bottom surface is inhibited whilst rolling of the non-agglutinated particles down the bottom surface is less inhibited.

The present invention also relates to an analyzing apparatus for immunological agglutinating reaction and has an object to provide a novel and useful apparatus which can effect an immunological analysis in a precise and efficient manner.

It is another object of the invention to provide an analyzing apparatus which can selectively identify various types of substances such as red blood cell type, white blood cell type, blood disc type and lymphocyte type and further can selectively detect various kinds of antigens, antibodies, viruses and specific proteins.

It is still another object of the invention to provide an analyzing apparatus which can be made simple in construction and small in size.

It is still another object of the invention to provide an analyzing apparatus in which a reaction time can be changed at will in a very simple manner.

According to another aspect of the present invention, there is provided an analyzing apparatus for agglutinating reaction comprising

means for feeding carriers each having therein a plurality of reaction vessels having inclined bottom surfaces, along a reaction line;

means for delivering given amounts of samples successively into the reaction vessels, while the carriers are fed along the reaction line;

means for delivering given amounts of reagents into the reaction vessels, while the carriers are fed along the reaction line;

means for detecting photoelectrically agglutination in the reaction vessels to produce detection signals; and

means for receiving the detection signals to effect an analysis through existence and non-existence of the agglutination;
characterized in that the feed means comprises means for feeding said carriers, when said carriers contain said samples and said reagents, along said reaction line in a steady manner and thus without subjecting said carriers to sharp shocks, in that the detecting means serves to detect agglutination patterns of particles on the inclined bottom surfaces of the reaction vessels, and in that the bottom surface of each reaction vessel is inclined to the horizontal in such a manner and is provided with a plurality of such steps that the motion of the agglutinated particles down the bottom surface is inhibited whilst rolling of the non-agglutinated particles down the bottom surface is less inhibited.

According to the invention the microplate having a number of reaction vessels is fed in a steady manner along the reaction line with respect to the sample delivering means, reagent delivering means and photoelectrically detecting means which are fixedly arranged. Contrary to this, in the known microtiter method, after the sample and reagent have been delivered, the reaction vessel must be kept perfectly stationary and thus, if this method is effected by an automatic analyzer, sample delivery means, reagent delivery means and detecting means have to be moved with respect to the stationary reaction vessels. Therefore, the analyzer is liable to be large in size and complicated in construction. The inventors have confirmed experimentally that even if the microplate is transported along the reaction line in a substantially stationary manner, the formation of agglutination pattern is hardly affected by movement and vibration, so that sufficiently clear and precise patterns can be obtained. Further, according to the invention, since use is made of the microplate having a number of reaction vessels, the mechanism for feeding the reaction vessels can be made extremely simple and further the processing capacity can be increased. In a preferred embodiment of the invention, the reaction line consists of a plurality of passages arranged in a zig-zag form. Such an apparatus can further be made small.

Moreover, each sample can be delivered into a plurality of reaction vessels and analyzing results can be derived by judging a plurality of agglutination patterns. Therefore very accurate analyses can be performed. When the apparatus is utilized in a transfusion center for identifying blood types of a very large number of blood samples, it is extremely important to increase the analyzing accuracy or precision. If misjudgement occurs, serious problems will arise.

An example of the invention will now be described in detail with reference to the accompanying drawings, wherein:

Fig. 1 is a schematic diagram for explaining successive steps of an analyzing method;

Fig. 2 is a schematic plan view illustrating an embodiment of the analyzing apparatus;

Fig. 3 is a flow chart showing successive operations of the analyzing apparatus of Fig. 2;

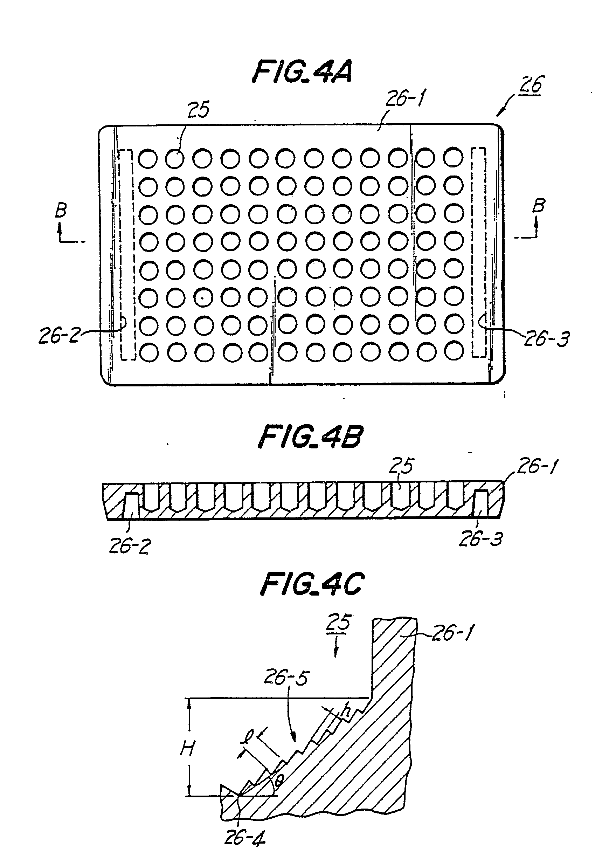

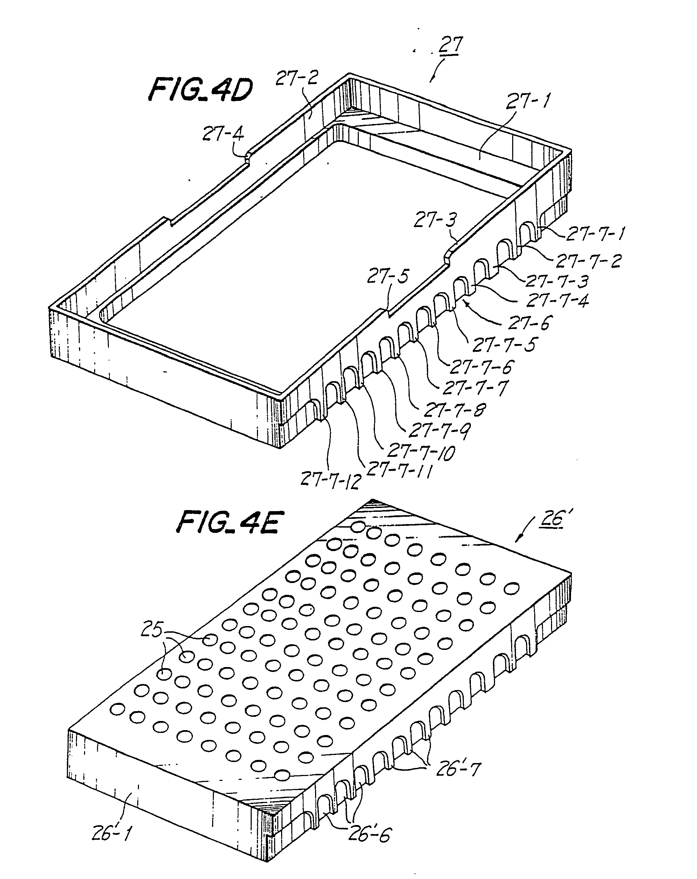

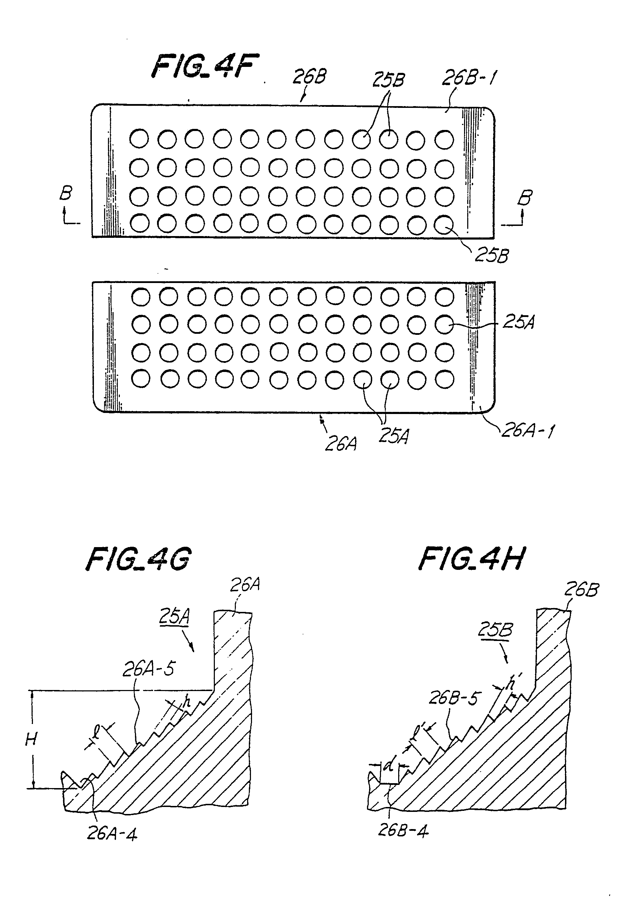

Fig. 4A is a plan view showing an embodiment of a microplate having a number of reaction vessels, Fig. 4B is a cross section cut along a line B—B in Fig. 4A, Fig. 4C is an enlarged cross section illustrating a configuration of a bottom surface of any one of the reaction vessels, Fig. 4D is a perspective view showing a cassette for holding the microplate, Fig. 4E is a perspective view depicting another embodiment of the microplate, Fig. 4F is a plan view showing still another embodiment of the microplate having microplate halves, Figs. 4G and 4H are enlarged cross sections illustrating bottom configurations of reaction vessels formed in the microplate halves;

Fig. 5A and 5B are schematic diagrams showing an embodiment of a sample diluting unit;

Fig. 6A is a perspective view illustrating an embodiment of a stopper for indexing the microplate, Figs. 6B to 6C are cross sections cut along a line B—B in Fig. 6A;

Figs. 7A to 7C show an embodiment of a pattern detecting unit;

Fig. 8 is a circuit diagram of an embodiment of a judging circuit;

Fig. 9 is a diagram showing a relation between blood types and agglutination patterns formed on the bottom surfaces of reaction vessels;

Figs. 10A and 10B are plan and side views illustrating an embodiment of a sample rack;

Figs. 11A and 11B are partially cut away plan and side views showing a rack cassette and an auto-loader;

Fig. 12 is a schematic diagram depicting an embodiment of a sample delivery unit;

Figs. 13 and 14 are plan and side views, respectively illustrating an embodiment of an auto-loader for supplying the microplate onto the reaction line;

Figs. 15, 16 and 17 are plan, front and side views, respectively showing an embodiment of a microplate transporting unit for moving the microplate between reaction line passages; and

Figs. 18A to 18C are diagrams showing a selective delivery of samples in different modes.

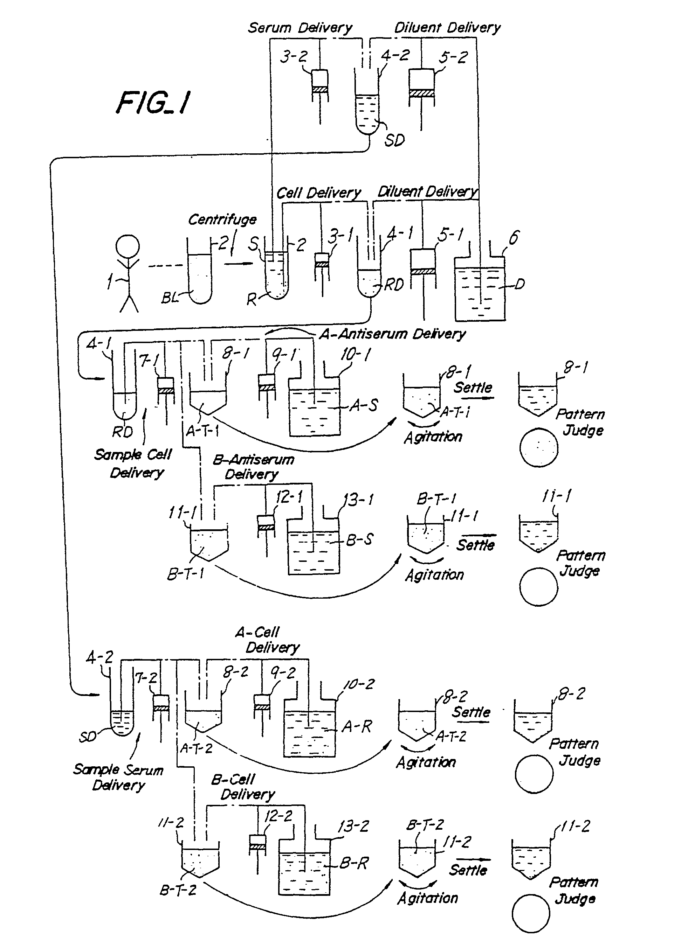

Fig. 1 is a schematic view showing successive steps of the analyzing method. A whole blood BL extracted from a patient 1 is contained in a test tube 2. The tube 2 is set in a known centrifuge and the whole blood BL is separated into a serum S and blood cells (red blood cells) R. In normal bloods, the blood cells constitute about 40%. Then 30 µl of blood cells R is aspirated by a delivering device 3-1 constituted by a microsyringe and is delivered into a test tube 4-1 into which 1,970 µl of a diluent D such as saline solution contained in a vessel 6 is also delivered by means of a delivering device 5-1. In this manner, in the tube 4-1, there is contained 2,000 µl of blood cell suspension RD of 1.5%. By means of a delivering device 3-2, 200 µl of the serum S in the tube 2 is delivered into a test tube 4-2 into which is also delivered by a delivering device 5-2, 600 µl of the diluent D so as to form 800 µl of a diluted serum SD of 25%.

Next, 25 µl of the blood cell suspension RD in the tube 4-1 is aspirated by a delivering device 7-1 and is discharged into a reaction vessel 8-1 having a conical surface. Into the reaction vessel 8-1 is delivered 25 µl of an A-type antiserum reagent A-S of 1.25% contained in a vessel 10-1 by means of a delivering device 9-1. Further, another 25 µl of the blood cell suspension RD in the tube 4-1 is delivered into a reaction vessel 11-1 by means of the delivering device 7-1 and 25 µl of a B-type antiserum B-S of 1.25% in a vessel 13-1 is also delivered into the reaction vessel 11-1 by a delivering device 12-1. In this manner, in the reaction vessels 8-1 and 11-1 there are obtained a test liquid A-T-1 which is a mixture of the blood cells to be tested and the A-type antiserum, and a test liquid B-T-1 which is a mixture of the blood cells to be tested and the B-type antiserum, respectively.

The diluted serum sample SD in the tube 4-2 is aspirated by a delivering device 7-2 by an amount of 25 µl and is delivered into a reaction vessel 8-2 into which is also delivered by a delivering device 9-2, 25 µl of an A-type blood cell reagent A-R of 1.2% contained in a vessel 10-2. Another 25 µl of the serum sample SD is delivered into a reaction vessel 11-2 by means of the delivering device 7-2. Into this vessel 11-2 is also delivered by a delivery device 12-2, 25 µl of a B-type blood cell reagent B-R contained in a vessel 13-2. In this manner, in the reaction vessels 8-2 and 11-2 there are formed a test liquid A-T-2 which is a mixture of the serum to be tested and the A-type blood cell reagent, and a test liquid B-T-2 which is a mixture of the sample serum to be tested and the B-type blood cell reagent, respectively. As explained later, the reaction vessels are integrally formed in the form of a microplate.

Then, the test liquids A-T-1, B-T-1, A-T-2 and B-T-2 contained in the reaction vessels 8-1, 11-1, 8-2 and 11-2, respectively are agitated by, for example, vibrating the vessels. In an embodiment of the analyzing apparatus according to the invention, when the antiserum reagents A-S, B-S and the blood cell reagents A-R, B-R are delivered into the reaction vessels 8-1, 8-2, 11-1 and 11-2, they are fully mixed with the sample blood cells RD and sample serums SD. In this case the separate agitation is no longer necessary.

Then the reaction vessels are moved along a reaction line slowly so that the test liquids are not subjected to hard shock, so that the reaction proceeds stably. During the reaction, the blood cells settle naturally due to gravity. A period of reaction may be, for example, thirty minutes. Now it is assumed that the sample blood is of A-type. Therefore, in the test liquid A-T-1 in which the A-type antiserum reagent A-S has been delivered, the blood cells are agglutinated and settle uniformly on the inclined bottom surface of the reaction vessel 8-1 in the manner of snow. Contrary to this, in the test liquid B-T-1 to which the B-type antiserum has been added, the blood cells are not agglutinated and roll down along the inclined bottom surface of the reaction vessel 11-1 and are collected at the lowest, center area of the bottom. In this manner, in case of agglutination, the blood cells settling on the inclined bottom surface of the vessel form a uniformly deposited pattern and in case of non-agglutination, the settling blood cells do not remain on the inclined surface, but are collected at the bottom center so as to form an integrated pattern. These patterns can be detected photoelectrically so as to judge the blood type. In general, the judgement thus effected with the aid of the sample blood cells is called a direct judgement. In order to form clear and precise integrated pattern in case of non-agglutination, the bottom surface of reaction vessel is preferably inclined by about 30° with respect to a horizontal plane.

In the reaction vessel 8-2, there is no agglutination and the integrated pattern is formed at the bottom center of vessel, while in the reaction vessel 11-2 the agglutination occurs so that the uniformly deposited pattern is formed on the bottom surface. By photoelectrically detecting these patterns it is also possible to judge the blood type. The judgement just explained above is generally referred to as indirect judgement. By effecting both the direct and indirect judgements, the type of the sample blood can be judged very accurately. The microplate having formed therein a number of reaction vessels in a matrix form is removed from the reaction line after the patterns formed on the bottoms of all of the reaction vessels have been detected.

In the above explanation, the reagents A-S, B-S, A-R and B-R are delivered into the reaction vessels after the blood cell and serum samples have been delivered. It should be noted that the order of delivery of these reagents and samples may be reversed or the reagents and samples may be delivered simultaneously.

In the blood type judging method explained above, since the reaction vessels are steady and the agglutination patterns, i.e. the uniformly deposited pattern and the integrated pattern are formed by natural sedimentation of blood cells, the blood type with incomplete antibodies having weak agglutinating force can be identified very accurately. Contrary to this, in the known method, the blood cells once agglutinated might be separated from each other by vibrating the reaction vessel. Further in the above method, the blood cell suspension can have a very small concentration such as 0.5 to 2% and each amount of suspension can be small such as 25 µl. Therefore, the amount of the sample blood can be very small. Contrary to this, in the known method, a great amount of blood cell suspension of 2 to 5% is required. It should be noted that the above method is not limited to determine the blood type of A, B, O-system, but may be equally applied to judge other blood types and to various immunological analyses.

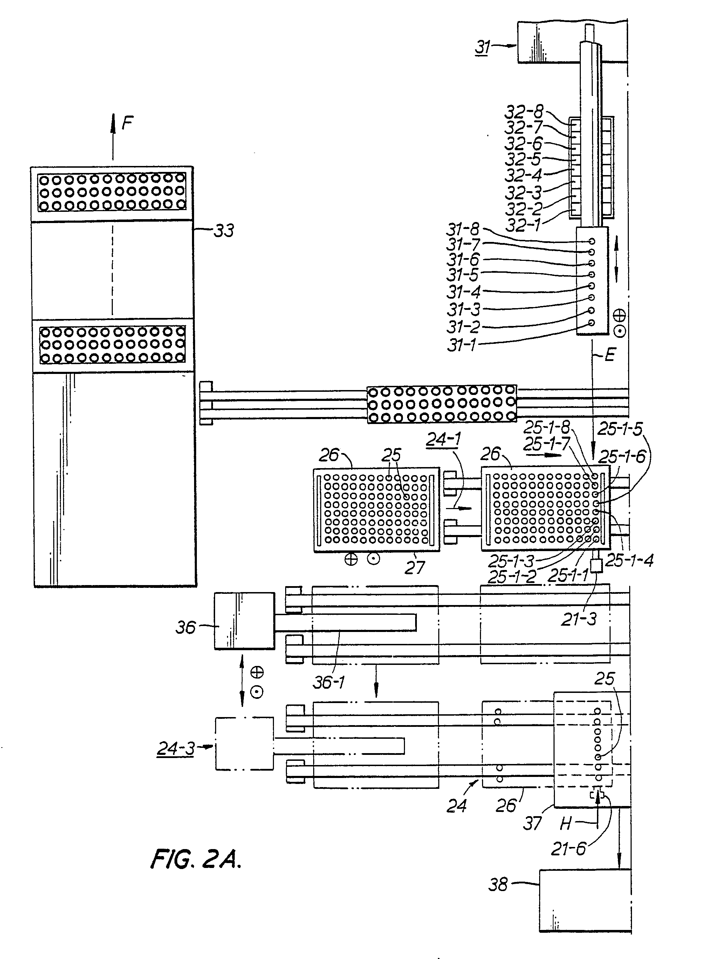

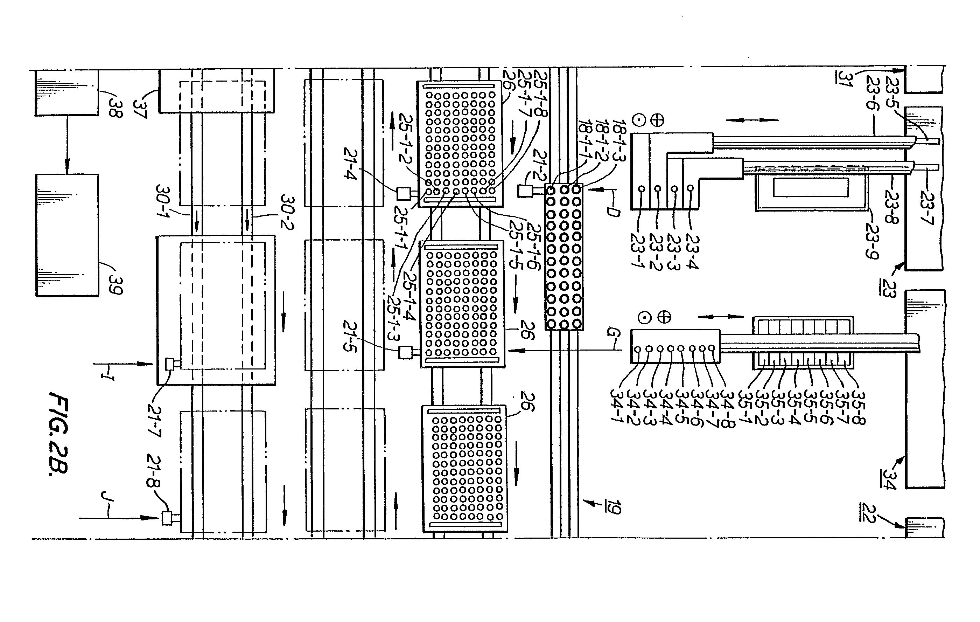

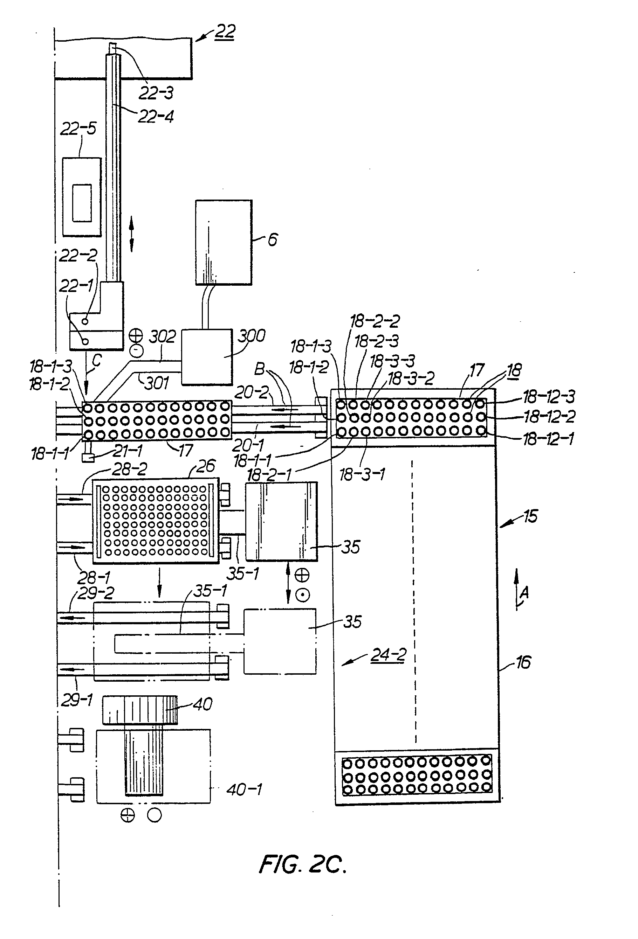

Fig. 2 is a schematic plan view showing an embodiment of the analyzing apparatus according to the invention. A reference numeral 15 generally denotes a sampler comprising a rack cassette 16 in which a number of racks 17 are set. In each rack are inserted a number of sample vessels or tubes 18. Three sample tubes 18-1, 18-2 and 18-3 are arranged in line and constitute a group. In a single rack 17, there are arranged twelve groups of sample tubes in parallel with each other. The lowermost tubes 18-1-1, 18-2-1, 18-3-1, ... 18-12-1 contain different sample bloods (whole bloods) which have been previously centrifuged, but the remaining sample tubes 18-1-2, 18-2-2, ... 18-12-2; 18-1-3, 18-2-3, ... 18-12-3 are vacant. The racks 17 containing the sample tubes 18 are fed in a direction shown by an arrow A at a given pitch and are successively positioned at an extension of a sample guide line 19. After a rack 17 has been transported into the sample guide line 19, the racks in the cassette 16 are simultaneously fed by one pitch in the direction A. Along the sample guide line 19 are arranged a pair of endless belts 20-1 and 20-2 which are always driven in a direction illustrated by arrows B at a relatively high speed. Therefore, the rack 17 transported onto the line 19 is fed leftward in Fig. 2. During the travelling of the rack 17 along the sample guide line 19, the rack is indexed at a sample diluting position C by means of a stopper 21-1. At this position C, given amounts of blood-cells and serum of the sample blood in the sample tube 18-1-1 are delivered into the vacant sample tubes 18-1-2 and 18-1-3, respectively by means of a delivery unit 22. The delivery unit 22 comprises two aspiration nozzles 22-1 and 22-2 connected to arms 22-3 and 22-4, respectively. These nozzles 22-1 and 22-2 are immersed in the sample blood contained in the tube 18-1-1 to different depths so as to be able to aspirate the blood cells and the serum, respectively. Then, the nozzles are raised out of the tube 18-1-1 and are moved horizontally to situate above the sample vessels 18-1-2 and 18-1-3, respectively. Then the aspirated blood cells and serum are discharged into the vessels 18-1-2 and 18-1-3, respectively. At the same time given amounts of a diluent, e.g. a saline solution contained in a diluent tank 6 are delivered into the sample tubes 18-1-2 and 18-1-3 by means of delivery probes 301 and 302, respectively so as to form a blood cell suspension of 1.5% and a diluted serum of 25%. Finally, the nozzles 22-1 and 22-2 are immersed in a washing tank 22-5 so as to wash the nozzles. After the above successive operations have been effected for the sample tubes 18-1-1, 18-1-2 and 18-1-3 in the first column in the rack 17, the stopper 21-1 is driven and three sample tubes 18-2-1, 18-2-2 and 18-2-3 in the second column in the rack 17 are indexed to the diluting position C and the same operations are effected for these tubes. In this embodiment, the sample tubes 18 in the rack 17 are intermittently fed at a period of fifteen seconds. Therefore, the delivering and diluting operations for all samples in the single rack 17 take a time equal to 15×12=180 seconds=3 minutes. The rack 17 is further fed in the direction B by means of the belts 20-1 and 20-2 and is then indexed at a sample delivery position D under the control of a stopper 21-2. At the sample delivery position D there is provided a sample delivery unit 23 comprising four delivery nozzles 23-1 to 23-4. Detailed construction and operation of the sample delivery unit will be explained later and thus, here only its function will be described.

As depicted in Fig. 2, the analyzing apparatus comprises a reaction line 24 including three reaction line passages 24-1 to 24-3 extending in parallel with the sample guide line 19 and the reaction vessels are fed along the reaction line passages in a zig-zag manner. A left-hand end of the first reaction line passage 24-1 is an entrance of the reaction line 24. From this entrance a cassette 27 containing a microplate 26 having formed therein a number of reaction vessels 25 is fed into the reaction line. The cassette and microplate will be further explained later with reference to Fig. 4. Further, for the sake of simplicity, a combination of the cassette 27 and microplate 26 is termed a microplate. In order to supply automatically the microplates successively into the reaction line 24, use may be made of various kinds of auto-loader. In the present embodiment, an auto-loader of elevator type is used in which a number of microplates 26 are stacked one on the other and are successively supplied into the reaction line 24 from the uppermost one. In Fig. 2 a microplate situated to the left of the passage 24-1 is not yet supplied onto the reaction line. In the reaction line passages 24-1, 24-2 and 24-3 there are provided pairs of endless belts 28-1, 28-2; 29-1, 29-2 and 30-1, 30-2, respectively. The belts 28-1, 28-2, 30-1 and 30-2 always travel in directions shown by arrows at a relatively high speed.

The microplate 26 supplied on the reaction line 24 is fed rightward by the belts 28-1 and 28-2 and is indexed at a reagent delivery position E by means of a stopper 21-3, said reagent delivery position being placed upstream with respect to the sample delivery position D viewed in the travelling direction of the microplate 26. In the present embodiment the microplate 26 comprises 8×12 reaction vessels 25 and thus, a plurality of different test items can be measured for respective samples. Among various test items, in this embodiment, the following items are to be analyzed.

1) direct judgement of ABO blood type
2) indirect judgement of ABO blood type
3) judgement of Rh blood type
4) antibody screening

That is to say, the direct judgement of ABO blood type is carried out with using first and second reaction vessels 25-1-1 and 25-1-2, the judgement of Rh blood type is effected by third and fourth reaction vessels 25-1-3 and 25-1-4, fifth and sixth reaction vessels 25-1-5 and 25-1-6 are used for the antibody screening and seventh and eighth reaction vessels 25-1-7 and 25-1-8 are employed for effecting the indirect judgement of ABO blood type. For this purpose, at the reagent delivery position E is provided a first reagent delivery unit 31 comprising eight delivery nozzles 31-1 to 31-8 for selectively delivering given amounts of reagents contained in reagent bottles 32-1 to 32-8. In the present embodiment, first to fifth bottles 32-1 to 32-5 contain an A-type antiserum of 12.5% (diluted with saline solution), a B-type antiserum of 12.5% (diluted with saline solution), a D-type antiserum of 12.5% (diluted with a buffer of phosphoric acid), a buffer of phosphoric acid and bromelin, respectively. At first, arms supporting the nozzles are moved inwardly so as to position the nozzles 31-1 to 31-8 just above the reagent bottles 32-1 to 32-8 and then the nozzles are immersed into the reagents to aspirate 25 μl of A-type antiserum, 25 μl of B-type antiserum, 12.5 μl of D-type antiserum, 12.5 μl of phosphoric acid buffer and a given amount of bromelin into the nozzles 31-1 to 31-5, respectively. Then the nozzles are removed from the reagent bottles and the arms are moved outwardly so as to position the nozzles 31-1 to 31-8 just above the reaction vessels 25-1-1 to 25-1-8, respectively situated at the first reagent delivery position E. Then each of the aspirated reagents are discharged into respective reaction vessels 25-1-1 to 25-1-5. In this manner, the reagent delivery operation for the first column of the reaction vessels has been completed, and then the stopper 21-3 is actuated to feed the microplate 26 by one pitch. After the reagent delivery for all columns of reaction vessels has been finished, the microplate 26 is fed rightward by means of the belts 28-1 and 28-2 and is indexed at the sample delivery position D by means of a stopper 21-4. At this position D, is provided the sample delivering unit 23 comprising the four nozzles 23-1 to 23-4 for delivering the blood suspension and diluted serum solution in the sample tubes 18-1-2 and 18-1-3, respectively into the reaction vessels 25-1-1 to 25-1-8. In the present embodiment, the blood cell sample in the tube 18-1-2 is aspirated into the nozzles 23-1 and 23-2 and the serum sample in the tube 18-1-3 is aspirated into the nozzles 23-3 and 23-4. The nozzles 23-1 to 23-4 are supported by arms 23-5 to 23-8. By suitably actuating the arms, the nozzles 23-1 to 23-4 are positioned above the reaction vessels 25-1-1, 25-1-3, 25-1-5 and 25-1-7, respectively. Then 25 µl of the blood sample of 1.5% aspirated in the nozzles 23-1 and 23-2 is discharged into the reaction vessels 25-1-1 and 25-1-3, respectively and 25 µl of the serum sample aspirated in the nozzles 23-3 and 23-4 is discharged into the reaction vessels 25-1-5 and 25-1-7, respectively. Then the arms 23-5 to 23-8 are further extended so as to situate the nozzles 23-1 to 23-4 just above the reaction vessels 25-1-2, 25-1-4, 25-1-6 and 25-1-8, respectively, and then 25 µl of the blood cell sample is discharged into the reaction vessels 25-1-2 and 25-1-4 and 25 µl of the serum sample is discharged into the reaction vessels 25-1-6 and 25-1-8. After that, the arms 23-5 to 23-8 are moved inwardly so as to immerse the nozzles 23-1 to 23-4 into a washing liquid in a washing tank 23-9.

After the blood cell sample and serum sample contained in the sample tubes 18-1-2 and 18-1-3 in the same column have been delivered into the reaction vessels 25-1-1 to 25-1-8 in the same column of the microplate 26 in the manner explained above, the stoppers 21-2 and 21-4 are actuated to move the rack 17 containing the sample tubes 18 leftward by one pitch and the microplate 26 including the reaction vessels 25 rightward by one pitch. The periods of these movements are identical and equal to 15 seconds, but the pitches differ from each other. In this manner the successive samples are delivered into successive reaction vessels and when the delivering operation for all samples contained in the single rack 17 has been completed, the rack 17 is moved leftwards by the belts 20-1 and 20-2 and is supplied into a rack-containing cassette 33. The cassette 33 is fed in a direction shown by an arrow F by a given pitch at a given period. When the sample delivery operation for all the reaction vessels in the single microplate 26 has been completed, the microplate 26 is moved rightward and is indexed at a second reagent delivery position G. At this position there is arranged a second reagent delivery unit 34 having entirely the same construction as that of the first reagent delivery unit 31. The second reagent delivery unit 34 comprises eight nozzles 34-1 to 34-8 correspondingly respectively to eight reagent bottles 35-1 to 35-8. In the present embodiment, the third to eighth reagent bottles 35-3 to 35-8 contain a bromelin solution of 0.44% (diluted with phosphoric acid buffer), a bromelin solution of 0.44% (diluted with phosphoric acid buffer), O-type blood cell reagent, O-type blood cell reagent, A-type blood cell reagent of 1.2% (diluted with saline solution) and B-type blood cell reagent of 1.2% (diluted with saline solution). 12.5 µl of bromelin is delivered into the reaction vessel 25-1-3 by means of the nozzle 34-3, 12.5 µl of bromelin is supplied into the reaction vessel 25-1-4 by means of the nozzle 34-4, given amounts of the O-type blood cell reagent are delivered into the reaction vessels 25-1-5 and 25-1-6 by means of the nozzles 34-5 and 34-6, respectively, 25 µl of A-type blood cell reagent is delivered into the reaction vessel 25-1-7 by means of the nozzle 34-7, and 25 µl of the B-type blood cell reagent is delivered into the reaction vessel 25-1-8 by means of the nozzle 34-8.

The second reagent delivery position G can be regarded as a reaction start position from which the agglutinating reaction is initiated and the microplate 26 is moved slowly for about thirty minutes. When the microplate 26 arrives at the right-hand end of the first reaction line passage 24-1, the microplate is stopped by a fixed stopper and is transported into a right-hand end of the second reaction line passage 24-2 by means of a microplate transporting unit 35 which will be explained later in detail. The transporting unit 35 comprises a plate-like arm 35-1 which can freely pass between the belts 28-1 and 28-2 and can be moved up and down in Fig. 2. At first the arm 35-1 is positioned below the first passage 24-1. After the microplate 26 has reached the right-hand end of the passage 24-1, the arm 35-1 is moved upward to arrest the microplate 26. The arm is further moved upward to lift the microplate above the belts 28-1 and 28-2. Then the arm 35-1 is moved horizontally toward the second passage 24-2 as shown by a chain line in Fig. 2. Then the arm 35-1 is moved downward between the belts 29-1 and 29-2 and during this movement the microplate 26 is arrested by the belts. In this manner, the microplate 26 can be transported from the end point of the first passage 24-1 to the start point of the second passage 24-2 and during this transportation the microplate 26 remains steady. In this embodiment the transportation takes three minutes.

In the second passage 24-2, the microplate 26 is fed leftward by means of the belts 29-1 and 29-2. Since any delivery is not effected in this passage 24-2, the belts 29-1, 29-2 may be moved at a very slow speed such as about 10 mm/15 sec. At an end point of the second passage 24-2 is arranged a fixed stopper and when the microplate 26 arrives at this point, it is transported into the third reaction line passage 24-3 by means of a second microplate transporting unit 36 comprising a plate-like arm 36-1, the construction and operation of the second transporting unit 36 being the same as those of the first transporting unit 35. In the third passage 24-3, the microplate 26 is fed right-ward by means of belts 30-1 and 30-2 which are driven at a relatively high speed and is first indexed at a pattern detecting position H by means of a stopper 21-6. At this position H, patterns of blood corpuscles formed on the bottom surfaces of the reaction vessels are detected photoelectrically by a pattern detection unit 37 which will be explained later. Since it takes thirty minutes for the microplate 26 to travel from the reaction start point G to the pattern detecting position H, the reaction proceeds for thirty minutes. An electrical signal is supplied from the pattern detection unit 37 to a judging circuit 38 which will be also explained later and in this circuit 38 judgements for various test items are effected and judged results are displayed by a display unit 39. At the pattern detecting position H, the microplate is fed at a given pitch corresponding to the columns of the reaction vessels in the microplate 26.

After the detection of patterns of all reaction vessels in the microplate 26 has been finished, the microplate is further fed rightward and is indexed at a photographing position I by means of a stopper 21-7. At this position I, an illumination lamp is arranged above the microplate and a camera is set below the microplate so as to take a photograph of the patterns of all the reaction vessels in the microplate simultaneously. After that the microplate 26 is further indexed at an inspecting position J by means of a stopper 21-8. At this position an operator can inspect with naked eyes the patterns formed on the bottom surfaces of the reaction vessels. For this purpose an illumination lamp is arranged underneath the microplate 26. When the microplate 26 arrives at the right-hand end of the third passage 24-3, the microplate is discharged into a microplate containing unit 40 in which successive microplates are stacked one on the other. The unit 40 comprises a plate 40-1 which moves up and down.

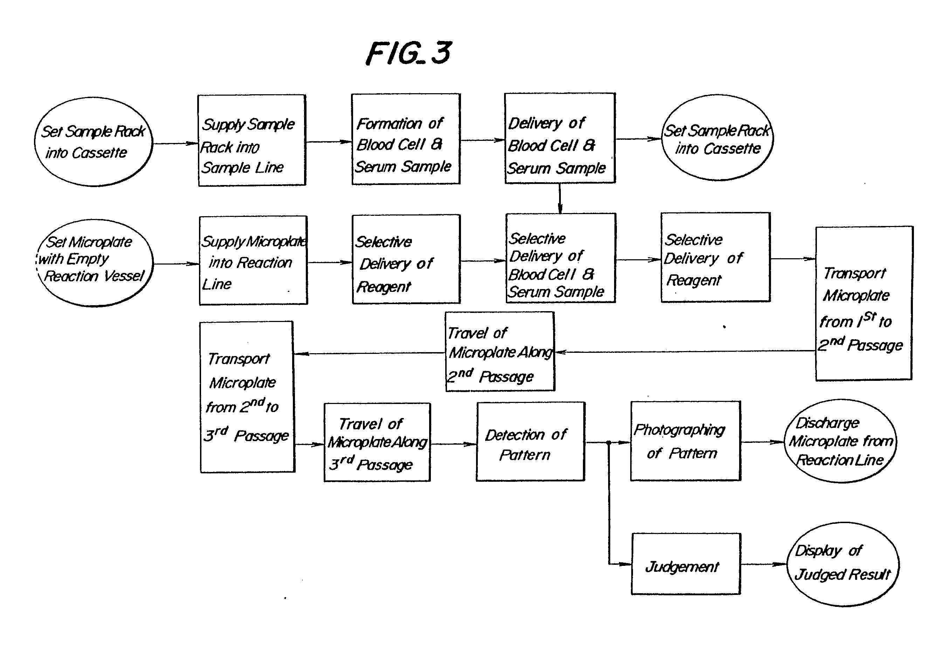

Fig. 3 is a flow chart showing successive operations of the analyzing apparatus shown in Fig. 2. Since the detailed operations can be clearly understood from the above explanation, a detailed description of the flow chart may be omitted.

Now the construction and operation of the various parts of the analyzing apparatus shown in Fig. 2 will be explained in detail.

Microplate

Fig. 4A is a plan view showing an embodiment of the microplate 26 and Fig. 4B is a cross sectional view cut along a line B—B in Fig. 4A. The microplate 26 comprises a substrate 26-1 made of transparent acrylic resin and having a length of 140 mm, a width of 84 mm and a thickness of 12 mm and 12×8 reaction vessels 25 each having a depth of 10 mm and a diameter of 6 mm. In the substrate, there are further formed recesses 26-2 and 26-3 from the underneath surface of the substrate, the recesses extending in a direction of the width of the substrate. The reaction vessel 25 has a conical bottom surface. As shown in Fig. 4C, in the inclined bottom surface of the reaction vessel there are formed a plurality of concentric steps 26-5 about a low bottom center 26-4. In this embodiment, the steps 26-5 are formed regularly over the entire bottom surface so that the cross sectional contour of the inclined bottom surface becomes a sawtooth shape. The vertical height H of the inclined bottom surface is 1.7 mm and the inclination angle θ of the bottom surface with respect to the horizontal plane is about 27°. A height h and a width l of the steps 26-5 are set to values within ranges of 2 to 50 μm and 5 to 200 μm, respectively depending upon size of settling particles. In the present embodiment, the steps have the same height and width as each other. It should be noted that height and width may be continuously decreased or increased from the bottom center 26-4 toward the periphery of the inclined bottom surface. Further, the steps may be formed by protrusions, depressions or a combination thereof. Further, the steps may be formed partially in the inclined bottom surface. With the steps in the bottom surface, a stable base layer of settled particles can be formed on the bottom surface and thus, the integrated pattern in case of non-agglutination and the uniformly deposited pattern in case of agglutination can be formed on the base layer in a clear and precise manner.

Fig. 4D is a perspective view illustrating an embodiment of the cassette 27 for holding the microplate 26. The cassette 27 has generally a frame configuration and comprises a frame-like bottom portion 27-1 on which the underneath surface of the microplate 26 is placed along its peripheral portion. The cassette 27 further comprises a pair of long side wall portions 27-2 and 27-3 in which notches 27-4 and 27-5 are formed for easily removing the microplate 26 out of the cassette 27. In an outer surface of one of the side wall portions 27-3 are formed a plurality of recesses 27-6 for indexing the microplate in conjunction with the stoppers 21-1 to 21-8. These recesses 27-6 are separated by twelve projections 27-7-1 to 27-7-12 corresponding to the number of the columns of the reaction vessels in the microplate 26.

It is not always necessary to set the microplate 26 into the cassette 27. Fig. 4E is a perspective view showing another embodiment of the microplate. In this microplate 26', a number of recesses 26'-6 and projections 26'-7 are formed in one side wall of the substrate 26'-1. Also in this embodiment, 8×12 reaction vessels 25 are integrally formed in the substrate 26'-1.

Fig. 4F is a plan view showing still another embodiment of the microplate. In this embodiment two microplate halves 26A and 26B comprising reaction vessels 25A and 25B, respectively are inserted in the cassette 27 shown in Fig. 4D. As shown in Figs. 4G and 4H, the reaction vessels 25A and 25B have different configuration and/or size. In the reaction vessel 25A in the microplate half 26A, steps 26A-5 having given height h and width l are formed over the whole bottom surface to a bottom center 26A-4, whereas in the reaction vessel 25B in the other microplate half 26B steps 26B-5 having given height h' and width l' are formed in part of the bottom surface and a bottom center 26B-4 is formed flat over a given diameter d. When a number of microplate halves are prepared and stocked, an optimum combination of the microplate halves can be selected in dependence upon test items to be performed. Of course it is possible to set in the cassette 27 two microplate halves having the same reaction vessels. Such a combination is entirely the equivalent of what is shown in Figs. 4A and 4D.

Sample delivery unit

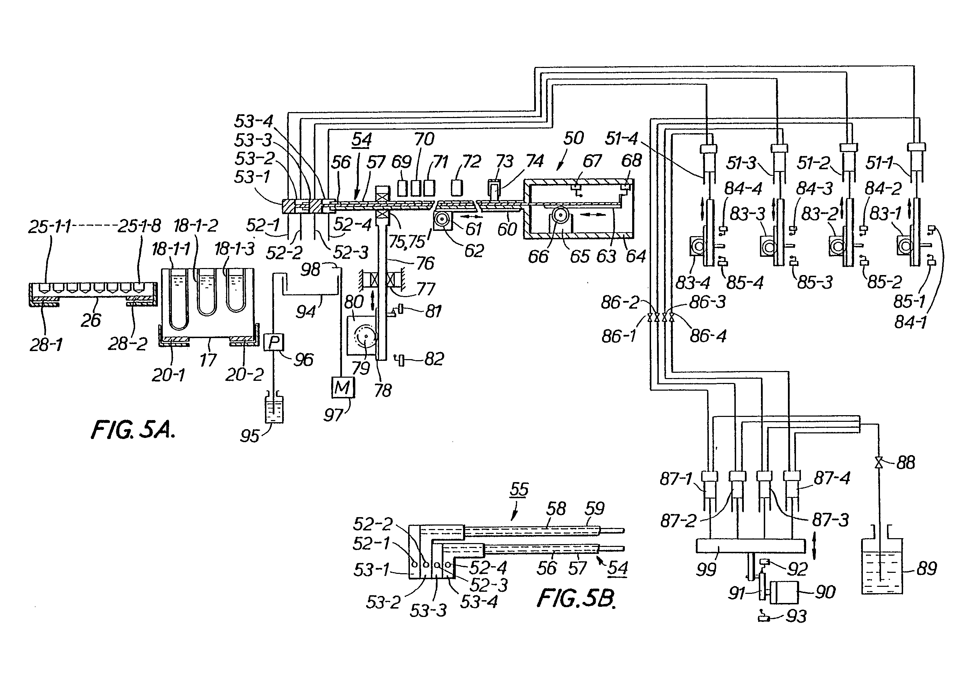

The sample delivery unit 50 (in Fig. 2 the same unit is denoted by the reference numeral 23) shown in Fig. 5A comprises four delivery nozzles 52-1 to 52-4 (in Fig. 2 they are shown by the reference numerals 23-1 to 23-4) and four syringes 51-1 to 51-4 connected to the nozzles 52-1 to 52-4, respectively. The syringes 51-1 to 51-4 can be operated independently from each other. The nozzles are aligned as clearly illustrated in Fig. 5B and are supported by supporting members 53-1 to 53-4, respectively. The members 53-3 and 53-4 are connected to a first feeding limb 54 and the members 53-1 and 53-2 are coupled with a second feeding limb 55. The first feeding limb 54 comprises an inner shaft 56 (in Fig. 2 denoted by the reference numeral 23-7) and an outer shaft 57 (in Fig. 2 represented by 23-8), the inner shaft 56 being slidably inserted in the outer shaft 57. By means of separate feeding mechanisms explained later, the inner and outer shafts 56 and 57 can be moved horizontally independently of each other. Similarly, the second feeding limb 55 comprises an inner shaft 58 (in Fig. 2 shown by 23-5) and an outer shaft 59 (in Fig. 2 denoted by 23-6). In this manner, the delivery nozzles 53-1 to 53-4 can be moved independently of each other by selectively moving the feeding shafts 56 to 59, respectively. In Fig. 5A, only the driving mechanism for the first feeding limb 54 is shown and a driving mechanism for the second feeding limb 55 may be constructed identically.

In the first feeding limb 55, the outer shaft 57 is moved horizontally in Fig. 5A by means of a rack 60 secured to the shaft 57, a pinion 61 engaged with the rack and a motor 62 coupled with the pinion 61. The inner feeding shaft 56 is driven by a rack 63 connected to that portion of the shaft 56 which protrudes beyond the outer shaft 57, a pinion 66 engaged with the rack 63 and a motor 65 connected to the pinion 66, the motor 65 being secured to a supporting member 64 which is movable together with the outer shaft 57. In the supporting member 64 are arranged a pair of switches 67 and 68 with a given interval for limiting the movable range of the inner shaft 56. Along the feeding limb 54 are arranged a plurality of photoswitches 69, 70, 71, 72 and 73 for setting operational positions of the outer shaft 57. The detection of the position of the outer shaft 57 is carried out by a shield plate 74 secured to the outer shaft 57 travelling between a light emitting element and a light receiving element installed in the respective photo-switches.

As explained above the second feeding limb 55 can be constructed in a similar manner. The first and second feeding limbs 54 and 55 are slidably supported by slide bearings 75 and 75' supported by a vertical feeding limb 76. The vertical feeding limb 76 is slidably supported by a slide bearing 77. To a lower end of the limb 76 is secured a rack 78 which is engaged with a pinion 79 connected to a motor 80. Then by driving the motor 80, the first and second feeding limbs 54, 55 are moved up and down. This up and down movement is limited by a pair of limit switches 81 and 82.

The syringes 51-1, 51-2, 51-3 and 51-4 connected to the nozzles 52-1, 52-2, 52-3 and 52-4, respectively can be independently operated by pulse motors 83-1, 83-2, 83-3 and 83-4, respectively via racks and pinions. Amounts of delivery can be simply and precisely controlled by adjusting the number of pulses supplied to the pulse motors. Movements of pistons of the syringes 51-1 to 51-4 may be limited by means of limit switches 84-1 to 84-4 and 85-1 to 85-4.

In order to wash pipes or tubes connecting the nozzles 52-1 to 52-4 to the syringes 51-1 to 51-4, washing syringes 87-1 to 87-4 are connected to the syringes 51-1 to 51-4 by means of valves 86-1 to 86-4. Suction tubes of the washing syringes 87-1 to 87-4 are commonly connected via a valve 88 to a washing liquid tank 89. The washing syringes 87-1 to 87-4 are simultaneously operated by a single operating device comprising a motor 90, a crank 91 and a rod 99 to which pistons of the syringes 87-1 to 87-4 are connected. Aspirating and discharging operations of washing liquid may be controlled by detecting a rotational angle of the crank 91 by means of microswitches 92 and 93. In this manner, the inner walls of nozzles 52-1 to 52-4 can be washed.

In order to wash the outer surfaces of nozzles 52-1 to 52-4, the front ends of the nozzles are moved downward into a washing tank 94 and then a washing liquid contained in a tank 95 is introduced in the tank 94 by means of a pump 96. After washing the nozzles are removed out of the tank 94 and during this movement air is projected onto the outer surfaces of the nozzles from an air nozzle 98 connected to a compressor 97 so as to scatter washing liquid drops applied on the nozzles. The washing liquid is continuously supplied into the washing tank 94, so that old, washing liquid overflows from the tank and the tank 94 is always filled with fresh washing liquid. The overflowed washing liquid is collected in a known manner.

Now the sample delivery operation will be explained in detail. In the following explanation various parts of the second feeding limb 55 are denoted by the same reference numeral with dash as those for denoting similar parts of the first feeding limb 54.

At first the delivery nozzles 52-1 to 52-4 are situated at stand-by positions shown in Fig. 5A. First of all the motors 62 and 62' are driven to move simultaneously the first and second feeding limbs 54 and 55 leftward. When the shield plate 74 arrives at the photo-switch 72, the motors 62, 62' are deenergized. At this time, it is assumed that the sample tubes 18-1-1, 18-1-2 and 18-1-3 in the first column in the rack 17 moving on the endless belts 20-1, 20-2 are indexed at the sample delivery position D. The delivery nozzles 52-1 and 52-2 are positioned above the sample tube 18-1-2 containing the blood cell sample and the nozzles 52-3 and 52-4 are situated above the sample tube 18-1-3 containing the serum sample.

Then the motor 80 is energized to move the first and second feeding limbs 54 and 55 downward until the nozzles 52-1 and 52-2 are immersed in the blood cell sample in the tube 18-1-2 and the nozzles 52-3 and 52-4 are immersed in the serum sample in the tube 18-1-3. Next, motors 83-1 to 83-4 are energized to aspirate given amounts of blood cell and serum samples into the nozzles 52-1, 52-2 and 52-3, 52-4, respectively. During this operation the valves 86-1 to 86-4 are all closed so as to prevent the washing liquid from being introduced into the syringes 51-1 to 51-4.

Then the motor 80 is driven in a reverse direction so as to move upward the first and second feeding limbs 54, 55, and then the delivery nozzles 52-1, 52-2 and 52-3, 52-4 are removed from the sample tubes 18-1-2 and 18-1-3, respectively.

Next, the motors 62 and 65 and motors 62' and 65' are operated in conjunction with each other and the nozzles 52-1 to 52-4 are positioned above given reaction vessels in the microplate 26 conveyed by the belts 28-1 and 28-2. The nozzle 52-4 secured to the supporting member 53-4 which is connected to the front end of outer shaft 57 is stopped at positions by means of the photosensors 69, 70 and 71. The nozzle 52-3 secured to the supporting member 53-3 connected to the front end of the inner shaft 56 is stopped at positions above the reaction vessels which are situated leftward with respect to the reaction vessels above which the nozzle 52-4 is stopped. The movement of the inner shaft 56 is controlled by the limit switches 67 and 68. The switches 67, 68 and photosensors 69 to 73 are so arranged that the nozzle 52-3 stops above the reaction vessel which is situated leftward by two vessels with respect to the reaction vessel above which the nozzle 52-4 is stopped. Similarly, the nozzle 52-2 supported by the member 53-2 secured to the front end of outer shaft 57' is indexed at positions under the control of the photosensors 69', 70' and 71', and the nozzle 52-1 supported by the member 53-1 secured to the front end of inner shaft 58 is positioned above the reaction vessel which is situated leftward by two vessels with respect to the reaction vessel above which the nozzle 52-2 is stopped.

Then the given amounts of aspirated blood cell sample and serum sample are discharged into the given reaction vessels by suitably actuating the syringes 51-1 to 51-4.

After delivery of the blood cell and serum samples into the reaction vessels, the motors 62, 65 and 62', 65' are energized in conjunction with each other so as to return the nozzles into a washing position (the stand-by position shown in Fig. 5A). The arrival of the nozzles at the washing position is detected by the photoswitches 73 and 73'. Then the feeding limbs 54, 55 are moved downward by energizing the motor 80 until the nozzles 52-1 to 52-4 are immersed in the washing liquid in the washing tank 94 so as to wash the outer surfaces of the nozzles. At the same time, the washing liquid is ejected from the nozzles 52-1 to 52-4 so as to wash the inner walls of the nozzles, connecting tubes and syringes 51-1 to 51-4. To this end the motor 90 is first energized to aspirate into the syringes 87-1 to 87-4 the washing liquid in the tank 89 via the valve 88, and then after the valve 88 is closed and the valves 86-1 to 86-4 are opened, the aspirated washing liquid is discharged from the syringes 87-1 to 87-4 into the washing tank 94 via the nozzles 52-1 to 52-4.

After washing, the motor 80 is energized in the reverse direction to move the nozzles upward. During this movement, the washing liquid drops applied on the nozzles are removed by projecting the air stream from the air nozzle 98. After the nozzles 52-1 to 52-4 are moved upward into the given position, the sample delivery operation is finished. According to the sample delivery unit 50 of the present embodiment, the two kinds of samples, i.e. the blood cell and serum samples can be delivered into the reaction vessels 25-1-1 to 25-1-8 in various manners by suitably driving the pulse motors 83-1 to 83-4 belonging to the syringes 51-1 to 51-4. In the above example, the blood cell and serum samples are delivered with a ratio of 4 to 4, i.e. the blood cell sample is delivered into four reaction vessels 25-1-1 to 25-1-4 and the serum sample is delivered into four reaction vessels 25-1-5 to 25-1-8. It should be noted that the blood cell and serum samples may be delivered into the eight reaction vessels by ratios of 5 to 3 and 6 to 2. In this manner, various kinds of test items can be effected.

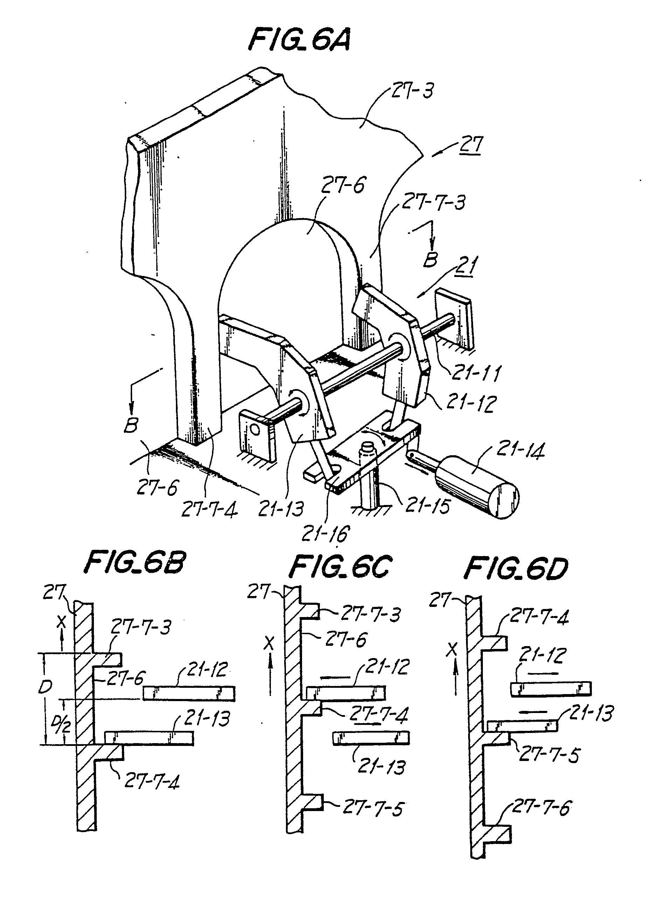

Fig. 6A is a perspective view showing an embodiment of the stopper 21 for indexing the microplate in position. The stopper comprises two claws 21-12 and 21-13 journalled to a shaft 21-11 extending in parallel with the side wall 27-3 of cassette 27. Free ends of the claws 21-12 and 21-13 are alternately projected into the recess 27-6 formed in the side wall 27-3 so as to engage with the projections 27-7-1 to 27-7-12. The other ends of claws 21-12 and 21-13 are coupled with opposite ends of a swingable plate 21-16 which is rotatably supported by a shaft 21-15 and the swingable plate 21-16 is coupled with a plunger of a solenoid 21-14.

Now the operation of the stopper 21 will be explained in detail with reference to Figs. 6B to 6D which are cross sectional views cut along a line B—B in Fig. 6A. In Figs. 6B to 6D, the cassette 27 is assumed to be moved as shown by an arrow X. In Fig. 6B, the solenoid 21-14 is deenergized and the claw 21-13 is projected into the recess 27-6 and is engaged with the projection 27-7-4, so that the cassette 27 is stopped. The distance between the claws 21-12 and 21-13 is equal to about a half of a pitch D of successive projections 27-7-3 and 27-7-4. When the solenoid 21-14 is energized for a given period to rotate the plate 21-16 in a clockwise direction, the claw 21-13 is removed from the recess 27-6 and the claw 21-12 is inserted into the same recess 27-6 as shown in Fig. 6C. Then the cassette 27 moves by about D/2 until the projection 27-7-4 is engaged with the claw 21-12. In this condition, the other claw 21-13 faces the next recess 27-6 formed by the projections 27-7-4 and 27-7-5. After a given time has elapsed, when the solenoid 21-14 is deenergized, the plate 21-16 is rotated in the anti-clockwise direction, so that the claw 21-12 is removed out of the recess 27-6 and the claw 21-13 is protruded into the next recess. Then the cassette 27 moves until the projections 27-7-5 is engaged with the claw 21-13 as illustrated in Fig. 6D. By repeating the operations shown in Figs. 6B to 6D it is possible to advance the cassette 27 and thus the microplate 26 pitch by pitch.

According to the present embodiment the solenoids of all the stoppers 21-1 to 21-8 can be simultaneously driven and thus, the timings of operations of the various parts of the analyzing apparatus can be controlled precisely in a simple manner. Further, by adjusting the period of the pulses to be supplied to the solenoids, the reaction time can be simply changed. For instance, when the period of the pulses is doubled, the reaction time becomes sixty minutes.

Pattern detecting unit

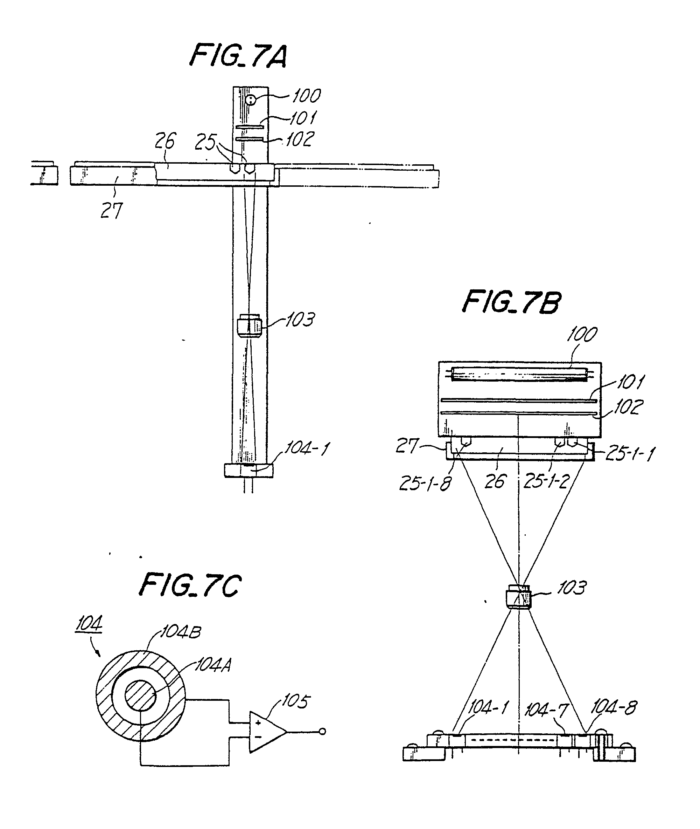

Figs. 7A to 7C show an embodiment of the pattern detecting unit 37 provided at the pattern detecting position H for detecting the corpuscle patterns formed on the bottom surfaces of reaction vessels 25 in the microplate 26. In the present embodiment, the patterns formed on the bottom surfaces of eight reaction vessels 25-1-1 to 25-1-8 in the column are simultaneously detected. To this end, above the microplate 26 is arranged an elongate illumination lamp 100 extending in the direction of width of the microplate. Light flux emitted from the lamp 100 is projected onto the microplate 26 by means of a diffusion plate 101 and a filter 102. Underneath the microplate 26 is arranged a projection lens 103 having a magnifying power of 1.5 to form images of the patterns formed on the bottom surfaces of the reaction vessels 25-1-1 to 25-1-8. These pattern images are received by eight light receiving elements 104-1 to 104-8, respectively. As illustrated in Fig. 7C each element 104 comprises two concentric light receiving regions 104A and 104B, the center region 104A receiving an image of a center of the pattern and the outer region 104B receiving an image of a peripheral portion of the pattern. It is now assumed that the sample blood to be tested is of A-type. When the A-type antiserum reagent is delivered into the reaction vessel, the agglutinating reaction is carried out and agglutinated blood cells are deposited on the inclined bottom surface as snow to form the uniformly deposited pattern. Then, the light flux impinging upon the regions 104A and 104B is partially shielded by the deposited particle layer and thus, amplitudes of signals supplied from the regions 104A and 104B have middle values which are substantially equal to each other. Therefore, a difference between the output signals from the regions 104A and 104B is small. Contrary to this, in the reaction vessel into which the B-type antiserum is delivered, no agglutination occurs and the integrated pattern is formed on its bottom surface. Therefore, the light flux directed at the center region 104A is greatly absorbed by the integrated particles and thus, the region 104A produces an output signal of small amplitude. On the other hand, the light flux directed at the peripheral region 104B is hardly shielded by the particles and thus the region 104B produces a large output signal. Therefore, the difference between the output signals from the regions 104A and 104B becomes very large. In this manner, by deriving the difference between the output signals from the regions 104A and 104B by means of a differential amplifier 105 as shown in Fig. 7C, it is possible to identify the pattern formed on the inclined bottom surface of the reaction vessel so as to judge the blood type of the sample blood.

Judging circuit

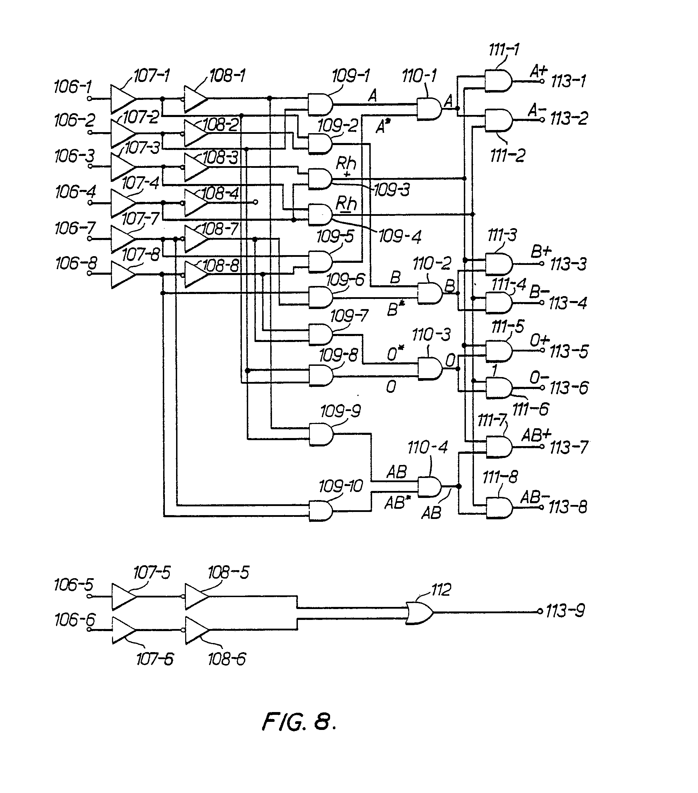

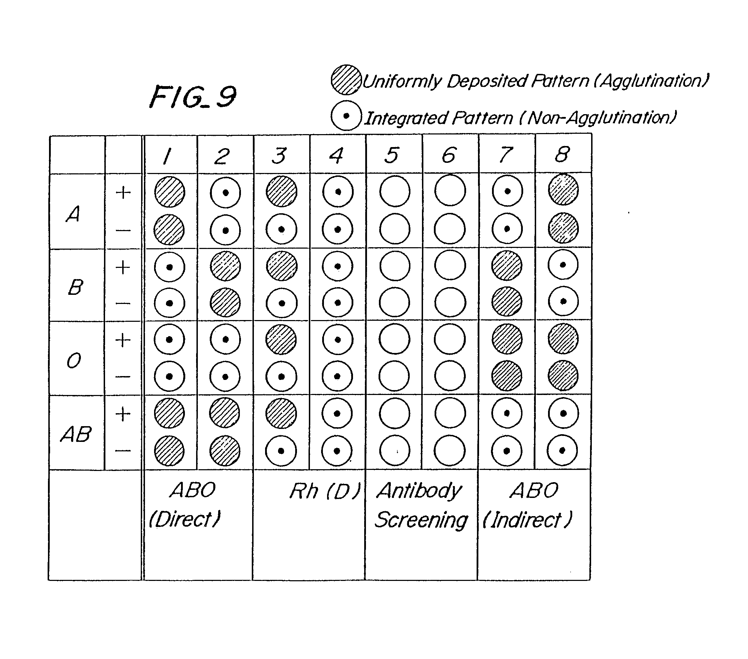

Fig. 8 is a circuit diagram showing an embodiment of the judging circuit 38. To input terminals 106-1 to 106-8 are supplied output signals from differential amplifiers receiving the output signals of the light receiving elements 104-1 to 104-8 shown in Fig. 7B. The judging circuit comprises amplifiers 107-1 to 107-8, inverters 108-1 to 108-8, AND gates 109-1 to 109-10, 110-1 to 110-4 and 111-1 to 111-8, and an OR gate 112. At output terminals 113-1 to 113-9, there may be obtained various judged results. These results can be clearly understood from Fig. 9 which shows relation between the patterns and the blood types. In Fig. 9, a hatched circle denotes the uniformly deposited pattern in case of agglutination and a circle with a center dot represents the integrated pattern in case of non-agglutination. In the reaction vessels Nos. 5 and 6, the antibody screening is effected and if either one of the vessels forms the uniformly deposited pattern, it is judged that the antibody is present. If an antibody is present in the reaction vessels, the agglutinating reaction is effected in either one or both of the reaction vessels 25-1-5 and 25-1-6 to form the uniformly deposited pattern. Therefore, by supplying the signals received at the input terminals 106-5 and 106-6 to the OR gate 112 via the inverters 108-5 and 108-6, respectively, there appears an output signal of high logic level (hereinafter referred to as H signal). Further, since the sample blood is of A-type, H signals are produced from the AND gates 109-1 and 109-5 and thus the AND gate 110-1 produces H signal. If the sample blood is of Rh$^+$, the AND gate 109-3 produces H signal and thus at the output terminal 113-1 is obtained H signal by means of the AND gate 111-1. The other signals will be supplied in a similar manner as shown in the following Table I. In Fig. 8, the indirect judgements are shown by adding asterisk * to letters A, B, O and AB denoting the blood types in ABO system and Rh$^+$ and Rh$^-$ are represented by adding signs + and −, respectively to the letters.

TABLE I

| Output terminal \ Blood type | A-type | | B-type | | O-type | | AB-type | |
|---|---|---|---|---|---|---|---|---|
| | Rh$^+$ | Rh$^-$ | Rh$^+$ | Rh$^-$ | Rh$^+$ | Rh$^-$ | Rh$^+$ | Rh$^-$ |
| 113-1 | H | L | L | L | L | L | L | L |
| 113-2 | L | H | L | L | L | L | L | L |
| 113-3 | L | L | H | L | L | L | L | L |
| 113-4 | L | L | L | H | L | L | L | L |
| 113-5 | L | L | L | L | H | L | L | L |
| 113-6 | L | L | L | L | L | H | L | L |
| 113-7 | L | L | L | L | L | L | H | L |
| 113-8 | L | L | L | L | L | L | L | H |

Sample rack

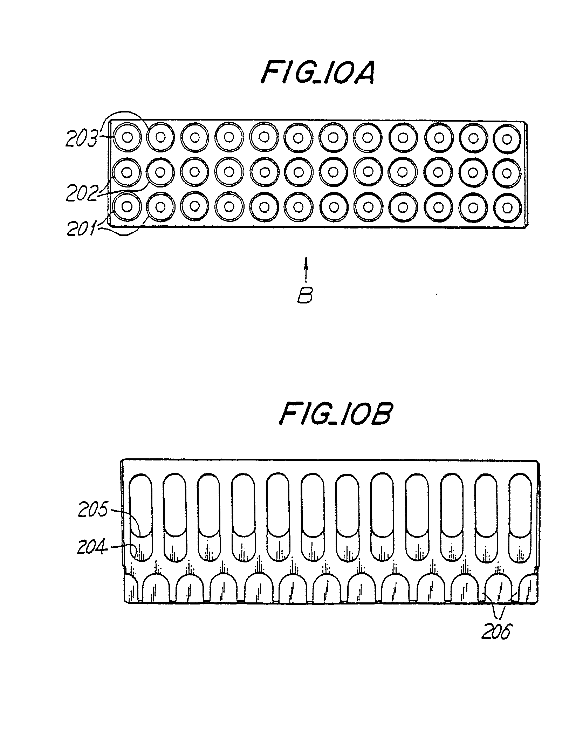

Fig. 10A is a plan view showing an embodiment of the sample rack 17 and Fig. 10B is a side view of the rack viewed from a direction B in Fig. 10A. In the rack 17 there are formed 3×12 holes 201, 202 and 203 for containing the sample tubes. In the holes 201 the sample tubes 18-1-1 to 18-12-1 containing the centrifuged blood samples are inserted, and in the holes 202 and 203 are inserted the sample tubes 18-1-2 to 18-12-2 and 18-1-3 to 18-12-3, respectively. In one side wall of the rack 17 are formed twelve openings 204 and in the other side wall are formed twelve openings 205. Through these openings, the operator can inspect the samples in the tubes. In the lower portions of said one side wall of rack 17 are also formed twelve projections 206 which are engaged with the stopper for feeding the rack 17 pitch by pitch.

Sampler

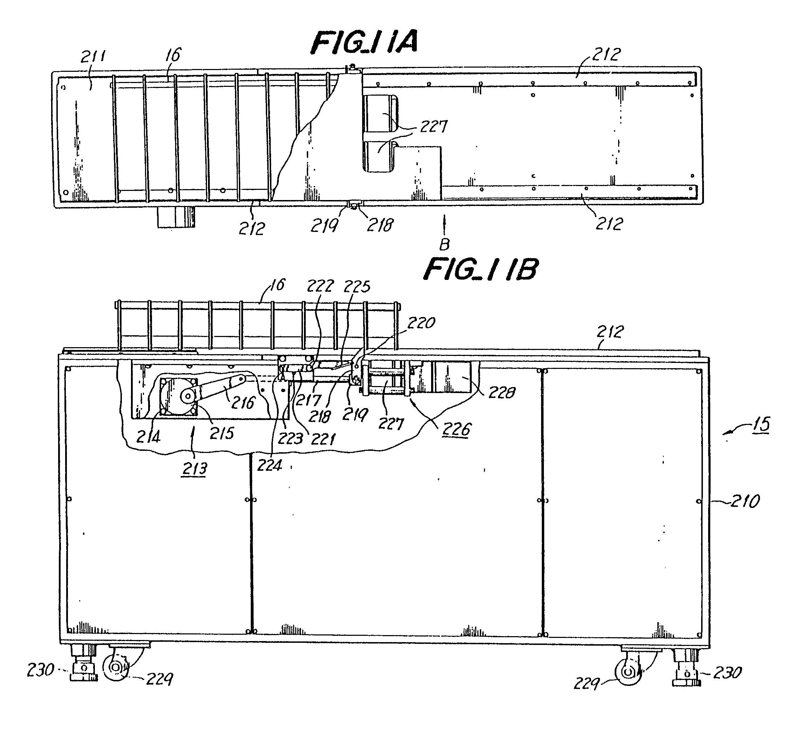

Fig. 11A is a plan view showing the sampler 15 and Fig. 11B is a partially cut away side view of the sampler viewed in a direction B in Fig. 11A. The sampler 15 comprises a housing or main body 210 and a slide plate 211 arranged on an upper part of the housing. On the slide plate 211 is provided a pair of guide rails 212. When the rack cassette 16 is placed on the sampler 15, the rack cassette 16 can slide on the slide plate 211 by means of the guide rails 212. The rack cassette 16 has seven sections for containing seven sample racks 17.

Underneath the slide plate 211 is arranged an intermittently feeding mechanism 213. The mechanism 213 comprises a motor 214 and a crank 215 connected to the motor directly or via a suitable decelerating mechanism. The rotational movement of the motor 214 is converted into a reciprocal movement by means of the crank 215 and this reciprocal movement is transferred to rods 216 and 217 so as to swing a claw 218 in a plane of Fig. 11B. The claw 218 is arranged to rotate only in the clockwise direction from the position shown in Fig. 11B. To this end a stopper member 219 is provided. The claw 218 is connected to a spring (not shown) for rotating the claw in the anticlockwise direction. To the claw 218 is further secured a pin 220 extending inwardly. The mechanism further comprises a guide lever 221 journalled to a shaft 222 and the lever 221 is biased by a spring 224 to rotate in the anticlockwise manner. This rotation is limited by a stopper pin 223. Therefore, the guide lever 221 can rotate in the clockwise direction against the force of the spring 224. At a free end of the guide lever 221 is formed a bent portion 225 which can be urged against the pin 220 on the claw 218.

When the motor 214 is energized to move the rod 217 rightward, the pin 220 is urged against an upper surface of the bent portion 225 and the lever 221 is rotated in the clockwise direction. Since the anticlockwise rotation of the claw 218 is limited by the stopper member 219, the top end of claw 218 is engaged with a protrusion of a separating plate of the rack cassette 16. Therefore, the rack cassette 16 is fed rightward. It should be noted that a stroke of the rod 217 and the claw 218 is set to be equal to one section in the rack cassette 16. Thus, the rack cassette 16 is fed by one section. When the rack cassette 16 has been fed by one section, the claw 218 has become disengaged from the bent portion 225 and thus the guide lever 221 is rotated in the anticlockwise manner due to the force of the spring 224. Fig. 11B shows this situation. Then the rod 217 and thus the claw 218 are moved leftward and the pin 220 is urged against the lower surface of the bent portion 225 of guide lever 221. Since the lever 221 cannot be further rotated in the anticlockwise direction, the claw 218 is rotated in the clockwise direction. Therefore, the claw 218 can return to the original position without engaging with the next separation plate of rack cassette 16. After the pin 220 has disengaged from the bent portion 225, the claw 218 is rotated in the anticlockwise direction due to action of the spring not shown. By repeating the above operations successively, it is possible to advance the rack cassette 16 intermittently section by section.

In the housing 210 there is further provided a rack feeding mechanism 226 comprising a pair of endless belts 227 and a motor 228. After the right-hand side rack in the rack cassette 16 has been fed on the belts 227 by means of the intermittently feeding mechanism 213, the motor 228 is energized at a controlled timing to feed the relevant rack in the direction B in Fig. 11A. In this manner, successive racks can be introduced in the sample line 19 in Fig. 2 at suitable timings.

The sampler 15 further comprises wheels 229 secured to a bottom plate, so that the sampler can be transported to a desired position easily. At this position, the sampler 15 can be fixedly settled by extending legs 230 by rotating them.

Sample diluting unit

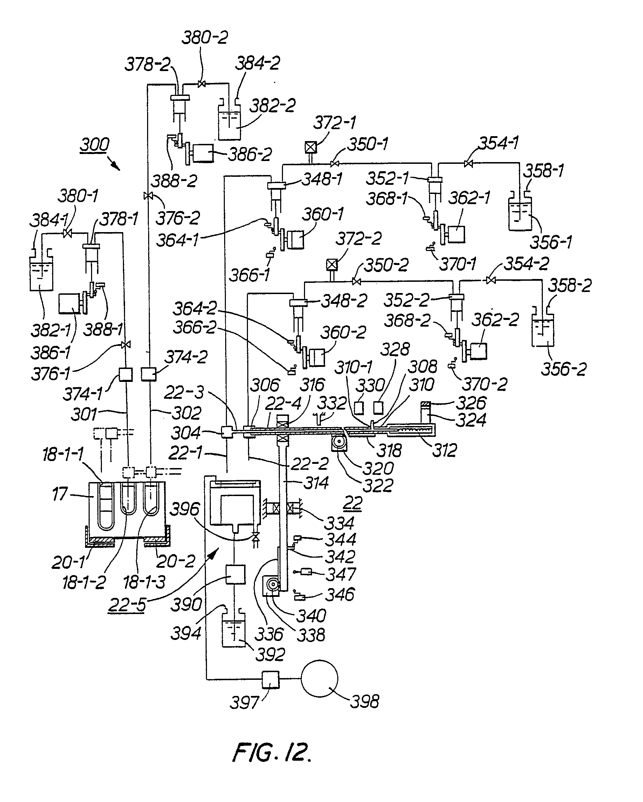

Fig. 12 is a schematic diagram showing an embodiment of the sample diluting unit comprising the sample delivery unit 22 and the diluent delivery unit 300. The sample delivery unit 22 comprises the two delivery nozzles 22-1 and 22-2 which are connected to the arms 22-3 and 22-4, respectively. In order to immerse the nozzles 22-1 and 22-2 into the blood cells and the serum in the sample tube 18-1-1, the nozzles have different length. That is to say, the nozzle 22-1 is longer than the nozzle 22-2. The arms 22-3 and 22-4 are provided coaxially in such a manner that the inner arm 22-3 can be slidably moved in the outer arm 22-4. At junctions between the nozzles and arms there are provided seals. At the rear end of the arm 22-3 is secured a projection 308 which is projected beyond the arm 22-4 through a slit-like opening 310 formed in the rear end of arm 22-4. Between the rear ends of the arms 22-3 and 22-4 is arranged a spring 312 for moving the arm 22-3 leftward in Fig. 12. This movement of the arm 22-3 is limited by an engagement of the projection with a left-hand side edge 310-1 of opening 310. The arm 22-4 is slidably supported by an upright limb 314 by means of a slide bearing 316, so that the arm 22-4 can be freely moved horizontally. A rack 318 is secured to the arm 22-4 and is engaged with a pinion 322 secured to a driving shaft of a motor 320. Then by rotating the motor 320 in both directions the arms 22-3, 22-4 can be moved rightward and leftward. At the rear end of the arm 22-4 is secured a shield plate 324 and photo-switches 326, 328 and 330 are arranged along a travelling path of the shield plate 324 so as to index the nozzles 22-1 and 22-2 at the washing, discharging and aspirating positions. Further in a travelling path of the projection 308 secured to the rear end of the arm 22-3 is arranged a stopper 332 for engaging with the projection so as to index the nozzle 22-1 at the aspirating position.

The limb 314 is slidably supported by a slide bearing 334. In order to move the limb 314 up and down, there are provided a rack 336 secured to the limb, a pinion 340 engaged with the rack and a motor 338 connected to the pinion. The up and down movement of the limb 314 and thus the arms 22-3, 22-4 and nozzles 22-1, 22-2 is limited by a projection 342 secured to the shaft and a pair of limit switches 344 and 346. Between these switches there is further arranged a third switch 347 for limiting the movement of the nozzle 22-1 in case of delivering only the serum sample by means of both the nozzles 22-1 and 22-2.

The nozzle 22-1 is connected via a syringe 348-1, a valve 350-1, a syringe 352-1 and a valve 354-1 to a tank 358-1 containing a washing liquid 356-1. The syringes 348-1 and 352-1 have pistons driven by pulse motors 360-1 and 362-1, respectively. Upper and lower dead points of the pistons are limited by microswitches 364-1, 366-1 and 368-1, 370-1, respectively. Around a tube between the nozzle 22-1 and the valve 350-1 is inserted a tube pinching device 372-1 for forming a small amount of an air layer at a tip of the nozzle 22-1 before aspiration.

Similarly, the nozzle 22-2 is communicated via a syringe 348-2, a valve 350-2, a syringe 352-2 and a valve 354-2 to a tank 358-2 containing a washing liquid 356-2. The syringes 348-2 and 352-2 are operated by means of pulse motors 360-2 and 362-2, respectively and microswitches 364-2, 366-2 and 368-2, 370-2 are provided for defining upper and lower dead points of pistons in the syringes 348-2 and 352-2, respectively. Further, a tube pinching device 372-2 is inserted between the nozzle 22-2 and the valve 350-2. It should be noted that fluid paths after the valves 354-1 and 354-2 may be common.

The diluent delivery unit 300 comprises two nozzles 301 and 302 arranged fixedly above the sample tubes 18-1-2 and 18-1-3, respectively. These nozzles 301 and 302 are communicated via dilution ratio selectors 374-1 and 374-2, valves 376-1 and 376-2, syringes 378-1 and 378-2 and valves 380-1 and 380-2 with tanks 384-1 and 384-2 containing washing liquids 382-1 and 382-2, respectively. The valves 376-1 and 380-1 and valves 376-2 and 380-2 are so constructed that when one of valves is opened, the other valve is closed. The syringes 378-1 and 378-2 have pistons actuated by pulse motors 386-1 and 386-2, respectively. Further, microswitches 388-1 and 388-2 are arranged to aspirate constant amounts of the washing liquid 382-1 and 382-2 into the syringes 378-1 and 378-2, respectively. Similar to the sample delivery unit 22, one of the tanks 384-1 and 384-2 may be omitted.

The washing tank 22-5 for washing the nozzles 22-1 and 22-2 is communicated via a pump 390 to a tank 394 containing a washing liquid 392. The used washing liquid in the tank 22-5 is discharged by means of a valve 396. At an upper part of the washing tank 22-5 is arranged an air nozzle (not shown) which is connected via a valve 397 to a compressor 398 so as to remove drops of washing liquid applied on the outer surface of the nozzles 22-1 and 22-2.

Now the operation of the delivery unit will be explained. In Fig. 12, the nozzles 22-1 and 22-2 are positioned above the washing tank 22-5. In this situation, the shield plate 324 is positioned at the photoswitch 326. Further the liquid paths connected to the nozzles 22-1 and 22-2 are filled with the washing liquids 356-1 and 356-2, respectively and air layers are formed in the tips of nozzles 22-1 and 22-2. When the motor 320 is energized to move the arms 22-3 and 22-4 leftward in Fig. 12, at first the projection 308 is engaged with the stopper 332 to stop the nozzle 22-1 at the sample aspirating position and after that only the arm 22-4 is moved leftward against the force of the spring 312. When the shield plate 324 comes to the photoswitch 330, the motor 320 is deenergized under the control of a signal from the photoswitch 330 to stop the nozzle 22-2 at the sample aspirating position. Then, the motor 338 is energized to move the limb 314 until the projection 342 actuates the microswitch 346. In this situation, the nozzles 22-1 and 22-2 are immersed into the blood cell sample and the serum sample, respectively in the sample tube 18-1-1. Then the pulse motors 360-1 and 360-2 are driven to aspirate given amounts of the blood cell and serum samples in the nozzles 22-1 and 22-2, respectively, while the valves 350-1 and 350-2 are closed.

After the aspiration, the motor 338 is driven in the reverse direction to lift the limb 314 and thus the nozzles 22-1 and 22-2 until the microswitch 344 is actuated. Then the motor 320 is driven in the reverse direction and only the arm 22-4 is moved rightward until the front edge 310-1 of opening 310 formed in the arm 22-4 comes into engagement with the projection 308. After that the arm 22-3 is moved rightward together with the arm 22-4. When the photoswitch 328 is actuated by the shield plate 324, the motor 320 is deenergized to stop the rightward movement of the arms 22-3 and 22-4. At this situation, the nozzles 22-1 and 22-2 are positioned just above the sample tubes 18-1-2 and 18-1-3, respectively. Then the motor 338 is driven again to move the upright limb 314 downward to insert the nozzles 22-1 and 22-2 into the tubes 18-1-2 and 18-1-3, respectively. After that the pulse motors 360-1 and 360-2 are driven to discharge the aspirated blood cell and serum samples into the tubes 18-1-2 and 18-1-3, respectively.

During a time in which the nozzles 22-1 and 22-2 travel from the washing position to the discharging position, the diluent delivery unit 300 is operated to aspirate in the syringes 378-1 and 378-2 given amounts of the diluents 382-1 and 382-2, respectively by closing the valves 376-1, 376-2, opening the valves 380-1, 380-2 and driving the motors 386-1, 386-2 for a given time period. Before discharging the blood cell and serum samples from the nozzles 22-1 and 22-2, respectively, the diluents are discharged from the syringes 378-1 and 378-2 through the dilution ratio selectors 374-1 and 374-2 and the nozzles 301 and 302 by driving the motors 386-1 and 386-2, while the valves 376-1, 376-2 are opened and the valves 380-1 and 380-2 are closed. A time period for discharging the diluent 382-1 from the nozzle 301-1 is set to be longer than a discharging period of the blood cell sample from the nozzle 22-1 which will be effected after initiation of the delivery of the diluent 382-1, and further the discharge of diluent 382-1 elapses after completion of the blood cell sample discharge. In other words, the blood cell sample is delivered into the sample tube 18-1-2 while the diluent 382-1 is discharged into the same sample tube. Therefore, the blood cells hardly adhere to the inner wall of the tube and are fully mixed with the diluent 382-1. If the discharge timing of the diluents 382-2 into the sample tube 18-1-3 is controlled in the same manner as that explained above, all the delivered serum sample can be fully mixed with the diluent 382-2.

After the blood cell and serum samples have been delivered into the sample tubes 18-1-2 and 18-1-3, respectively, the motor 338 is energized to lift the nozzles 22-1 and 22-2 by means of the upright limb 314. Then, the motor 320 is driven to move the arms 22-3 and 22-4 rightward until the shield plate 324 actuates the photoswitch 326. In this manner the nozzles 22-1 and 22-2 are positioned just above the washing tank 22-5. Now the motor 338 is energized again to move downward the arms 22-3 and 22-4 via the limb 314 until the nozzles 22-1, 22-2 are immersed into the washing tank 22-5. Then the valve 396 is closed and the pump 390 is driven to supply the washing liquid 392 into the washing tank 22-5. At the same time, the washing liquids 356-1 and 356-2 are discharged into the tank 22-5 through the nozzles 22-1 and 22-2, respectively by opening the valves 350-1, 350-2, closing the valves 354-1, 354-2 and driving the pulse motors 362-1, 362-2. It should be noted that the washing liquids 356-1, 356-2 have been previously aspirated into the syringes 352-1, 352-2 by closing the valves 350-1, 350-2, opening the valves 354-1, 354-2 and driving the pulse motors 362-1, 362-2. In this manner, both the inner and outer surfaces of the nozzles 22-1, 22-2 can be fully washed and any contamination between successive samples can be effectively avoided. After washing, the washing liquid in the tank 22-5 is discharged by opening the valve 396. At the same time, the motor 338 is energized again to move the nozzles 22-1 and 22-2 upwards. During this upward movement, the compressor 398 is energized to eject the air stream from the air nozzle not shown to blow off the washing liquid applied on the outer surfaces of the nozzles 22-1, 22-2. Further, the tube pinching device 372-1, 372-2 are driven for a short time to form the air layers in the tips of nozzles 22-1, 22-2 to prepare a next delivery operation.

In the sample delivery unit 22 shown in Fig. 12, the syringes 348-1 and 348-2 are driven by the pulse motors 360-1 and 360-2, respectively and thus, the amounts of the blood cell and serum samples can be simply changed by adjusting the numbers of driving pulses to be supplied to the pulse motors 360-1, 360-2.

Further, in the diluent delivery unit 300, the amounts of the diluents 382-1, 382-2 to be delivered into the sample tubes 18-1-2, 18-1-3 can be set at will by the dilution ratio selectors 374-1, 374-2 and thus, the blood cell suspension and serum solution having any desired concentrations can be formed simply.

Microplate auto-loader

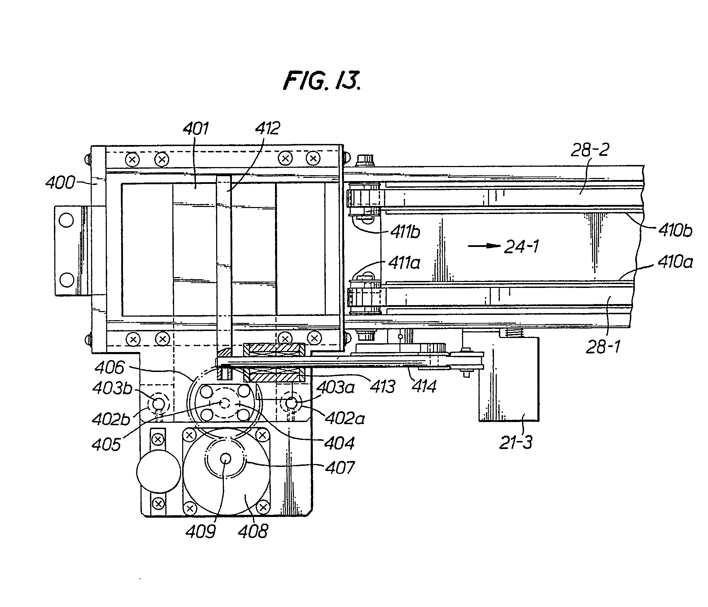

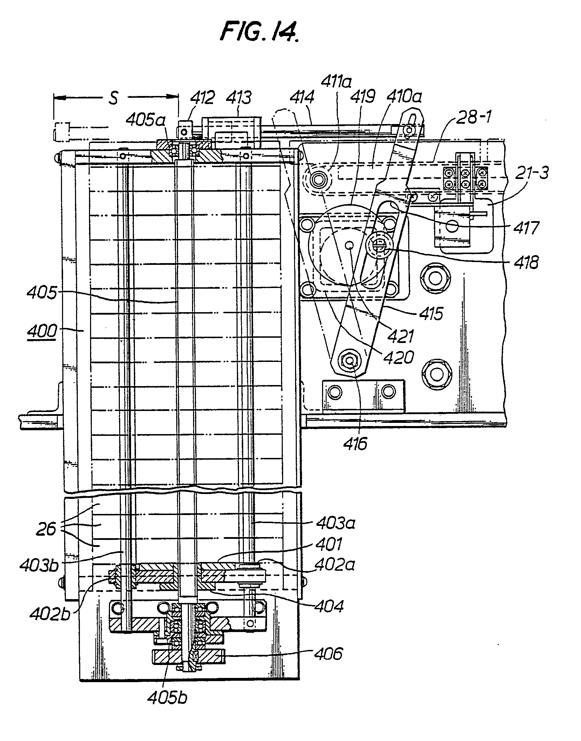

Figs. 13 and 14 are plan and side views illustrating the microplate auto-loader for automatically supplying the microplates into the entrance of the first reaction line passage 24-1. A number of microplates 26 are stacked one on the other in a frame like container 400. At the bottom of container 400 is arranged a bottom plate 401 which can be moved up and down. To an extension of the bottom plate 401 are secured bearings 402a and 402b which guide it on uprights shafts 403a and 403b, respectively. To the bottom plate 401 is also secured a nut 404 which is engaged with a lead screw 405. Therefore, by rotating the lead screw 405, the bottom plate 401 can be moved up and down. To this end, the lead screw 405 is journalled at its upper and lower ends in bearings 405a and 405b. To the lower portion of the screw 405 is fixed a first gear 406 which is engaged with a second gear 407 coupled to a driving shaft 409 of a reversing motor 408. Therefore, by driving the motor in forward and backward directions, the bottom plate 401 is moved upward and downward. In order to feed the microplate into the first reaction line path 24-1, the motor 408 is intermittently driven in the forward direction to move upward the bottom plate 401 by a given distance equal to the thickness of microplate 26.

Now the operation of the microplate autoloader will be explained in detail. As illustrated in Fig. 13, the passage 24-1 comprises a pair of endless belts 28-1 and 28-2 and guide rails 410a and 410b arranged immediately below the belts 28-1 and 28-2, respectively. The belts are rotated by means of rollers 411a and 411b and a motor not shown. The uppermost microplate contained in the container 400 has a bottom surface which is at or slightly higher than the level of the belts 28-1, 28-2. In order to push this microplate onto the belts, there is provided a pushing rod 412 which is attached to one end of a connecting rod 414 supported movably in a horizontal plane by a bearing 413. The other end of the rod 414 is articulated to one end of a lever 415, the other end of the lever being mounted on a fixed member by means of a pivot 416. In the middle of lever 415 is formed an elongate hole 417 through which a pin 418 extends. The pin 418 is secured to a disc 419 which is coupled with a driving shaft 421 of a motor 420. Therefore, by driving the motor 420 in one direction, the pushing rod 412 can be reciprocated in the horizontal plane so as to feed the uppermost microplate in the container 400 into the entrance of the first reaction line passage 24-1. After return of the pushing rod 412 into the initial position, the motor 408 is energized to move upward the bottom plate 401 by the given pitch equal to the thickness of microplate. As shown in Fig. 14, a stroke S of the pushing rod 412 is shorter than the length of microplate 26 and thus, at first the microplate is partially placed on the belts 28-1 and 28-2. After that, the microplate is fed by the belts.

Microplate transporting unit

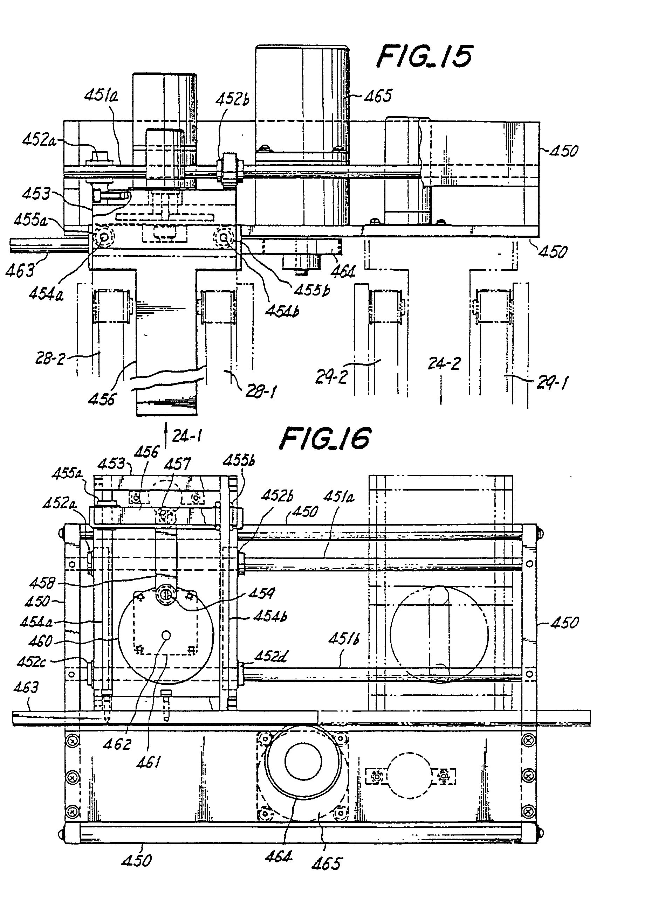

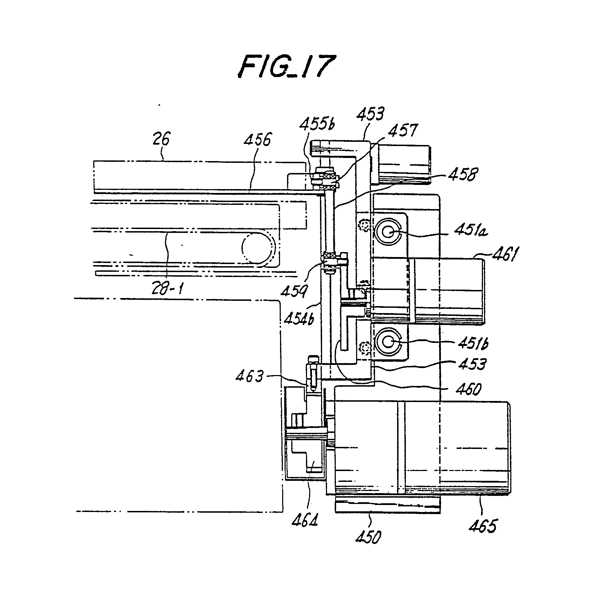

Now the microplate transporting unit 35 for conveying the microplate from the first reaction line passage 24-1 to the second reaction line passage 24-2 will be explained in detail with reference to Figs. 15, 16 and 17. The unit comprises a base 450 to which are secured a pair of rods 451a and 451b extending in the horizontal plane in a direction perpendicular to the passages 24-1, 24-2. On the rods 451a, 451b is slidably mounted a carriage 453 by means of bearings 452a to 452d. To the carriage 453 are secured a pair of upright rods 454a and 454b, and a lift plate 456 is slidably mounted on the rods 454a and 454b by means of bearings 455a and 455b. Therefore, the lift plate 456 can be moved vertically as well as horizontally. To the lift plate 456 is secured a pin 457 which is articulated to one end of a link 458, the other end of which is articulated to a disc 460 by means of a pin 459. The disc 460 is connected to a driving shaft 462 of a motor 461 secured to the carriage 453. Therefore, by rotating the motor 461, the lift plate 456 is moved up and down. In order to move the carriage 453 horizontally along the rods 451a and 451b, there are provided a rack 463 secured to the bottom of carriage 453, a pinion 464 engaged with

**0 051 496**

the rack and a reversing motor 465 coupled with the pinion.

Next, the operation of the microplate transporting unit 35 will be explained. It is assumed initially that the lift plate 456 is situated at a position below the first reaction line passage 24-1. When the microplate is fed into the end position of the first passage 24-1 by means of the belts 28-1 and 28-2, the motor 461 is driven at a suitable timing so as to rotate the disc 460 by 180° to move the lift plate 456 upward. During this upward movement, the microplate 26 is arrested by the lift plate 456 and is moved above the belts 28-1, 28-2. This situation is illustrated in Fig. 16. Then, the motor 465 is driven in the forward direction to rotate the pinion 464 in the clockwise direction in Fig. 16 and thus, the carriage 453 and the lift plate 456 are moved rightward until the lift plate is positioned above the second passage 24-2. Then the motor 461 is energized again to rotate the disc 460 by 180° to move the lift plate 456 downward. During this downward movement, the microplate 26 is arrested by the belts 29-1 and 29-2 of the second path. The lift plate 456 is further moved downward below the belts 29-1, 29-2. Next, the motor 465 is driven in the reverse direction to move the carriage 453 and thus the lift plate 456 leftward in Fig. 16 until the lift plate 456 returns to the initial position underneath the first reaction line path 24-1. By repeating the above operations, successive microplates can be transported from the first passage 24-1 to the second passage 24-2.

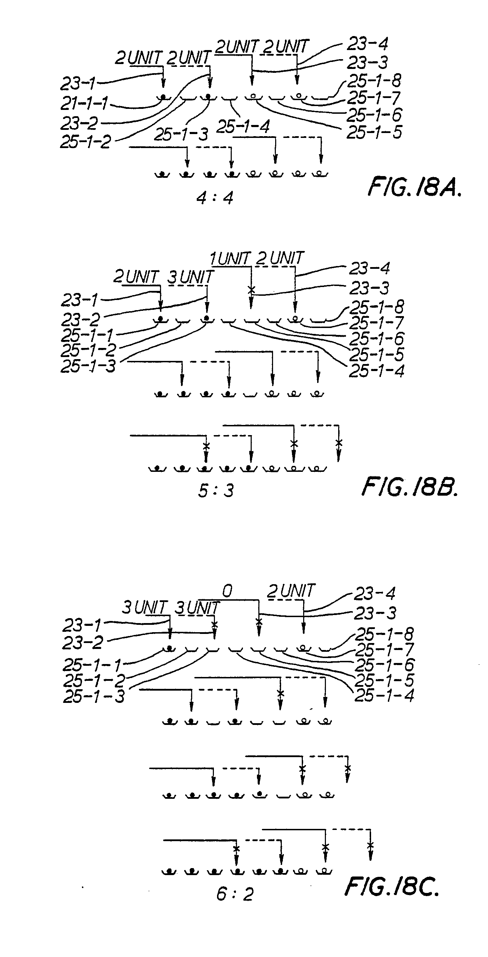

In the sample delivery unit 23 explained above, the blood cell sample is delivered into the four reaction vessels among the eight reaction vessels in the column and the serum sample is delivered into the remaining four reaction vessels by means of four sample delivery nozzles 23-1 to 23-4. It should be noted that the different kinds of samples may be delivered into the reaction vessels 25-1-1 to 25-1-8 in various modes. Figs. 18A to 18C are schematic diagrams illustrating a few examples of the delivery modes. In Fig. 18A, each of two kinds of samples are delivered into respective four reaction vessels. The samples have been aspirated into the nozzles 23-1, 23-2 and 23-3, 23-4, respectively by two units (an amount of sample to be delivered into a reaction vessel is termed one unit). At first, one unit of the first sample is delivered into each of the vessels 25-1-1 and 25-1-3 from the nozzles 23-1 and 23-2, respectively, and at the same time one unit of the second sample is delivered each of into the reaction vessels 25-1-5 and 25-1-7 from the nozzles 23-3 and 23-4, respectively. Then the nozzles 23-1 to 23-4 are moved rightward by one pitch and single units of the first and second samples are delivered into the reaction vessels 12-1-2, 12-1-4 and 12-1-6, 12-1-8, respectively from the nozzles 23-1, 23-2 and 23-3, 23-4. In this manner the samples are delivered in the eight vessels with 4 to 4 mode.

In an example shown in Fig. 18B, the first sample has been aspirated into the nozzles 23-1 and 23-2 by two and three units, respectively and the second sample has been aspirated into the nozzles 23-3 and 23-4 by one and two units, respectively. At first, single units of the first and second samples are delivered into the reaction vessels 12-1-1, 12-1-3 and 12-1-7, respectively from the nozzles 23-1, 23-2 and 23-4. In Fig. 18B a cross mark x denotes that no delivery action is effected. Then the nozzles 23-1 to 23-4 are moved rightward by one pitch, and the first and second samples are delivered into the vessels 12-1-2, 12-1-4 and 12-1-6, 12-1-8, respectively from the nozzles 23-1, 23-2 and 23-3, 23-4, respectively. Next, the nozzles 23-1 to 23-4 are further moved rightward by one pitch, and the first sample is delivered into the remaining vessel 12-1-5. Therefore, in this delivery mode, the first and second samples are delivered with 5 to 3.

Fig. 18C illustrates still another example of sample delivery, in which the first and second samples can be delivered at a ratio of 6 to 2. As shown in Fig. 18C, the first sample has been aspirated into each of the nozzles 23-1 and 23-2 by three units, respectively, and the second sample has been aspirated only into the nozzle 23-4 by two units. By discharging the samples into the reaction vessels 12-1-1 to 12-1-18 in three steps, it is possible to deliver the first and second samples at a ratio of 6:2. It should be noted that only the single kind of sample may be delivered into the eight reaction vessels by any desired amounts. In this manner various combinations of many test items may be selectively formed. Further, since the concentration of the reagents can be adjusted in various manners in combination of the sample diluting unit, a quite large number of combinations of the test items can be performed. Some of the combinations will be shown in the following Tables II to V.

TABLE II

| Blood type | Reaction vessel | 1st Reagent | Sample | | 2nd Reagent |
|---|---|---|---|---|---|
| ABO-type (direct) | 25-1-1 | A-type antiserum diluted with saline solution 12.5%    25 µl | blood suspension diluted with saline solution 1.5%    25 µl | (a) | |
| | 25-1-2 | B-type antiserum diluted with saline solution 12.5%    25 µl | blood suspension diluted with saline solution 1.5%    25 µl | (a) | |
| Rh-type | 25-1-3 | D-type antiserum diluted with phosphoric acid 25%    12.5 µl | blood suspension diluted with saline solution 1.5%    25 µl | (a) | bromelin diluted with phosphoric acid 0.44%    12.5 µl |
| | 25-1-4 | phosphoric acid 12.5 µl | blood suspension diluted with saline solution 1.5%    25 µl | (a) | bromelin diluted with phosphoric acid 0.44%    12.5 µl |
| Antibody screening | 25-1-5 | bromelin | serum diluted with saline solution 25% | (b) | O-type blood cell reagent processed with bromelin |
| | 25-1-6 | | serum diluted with saline solution 25% | (b) | O-type blood cell reagent |
| ABO-type (indirect) | 25-1-7 | | serum diluted with saline solution 25%    25 µl | (b) | A-type blood cell reagent diluted with saline solution 1.2%    25 µl |
| | 25-1-8 | | serum diluted with saline solution 25%    25 µl | (b) | B-type blood cell reagent diluted with saline solution 1.2%    25 µl |

0 051 496

19

TABLE III

| Blood type | Reaction vessel | 1st Reagent | Sample | | 2nd Reagent |
|---|---|---|---|---|---|
| ABO-type (direct) | 25-1-1 | A-type antiserum diluted with saline solution 12.5%   25 µl | blood suspension diluted with saline solution 1.5%   25 µl | (a) | |
| | 25-1-2 | B-type antiserum diluted with saline solution 12.5%   25 µl | blood suspension diluted with saline solution 1.5%   25 µl | (a) | |
| Rh-type | 25-1-3 | D-type antiserum diluted with bromelin phosphoric acid 25%   12.5 µl | blood suspension diluted with saline solution 1.5%   25 µl | (a) | |
| | 25-1-4 | bromelin | blood suspension diluted with saline solution 1.5%   25 µl | (a) | |
| Antibody screening | 25-1-5 | dolichos 25 µl | blood suspension diluted with saline solution 1.5%   25 µl | (a) | |
| | 25-1-6 | | serum diluted with saline solution 25%   25 µl | (b) | O-type blood cell reagent 25 µl |
| ABO-type (indirect) | 25-1-7 | | serum diluted with saline solution 25%   25 µl | (b) | A-type blood cell reagent diluted with saline solution 1.2%   25 µl |
| | 25-1-8 | | serum diluted with saline solution 25%   25 µl | (b) | B-type blood cell reagent diluted with saline solution 1.2%   25 µl |

TABLE IV

| Blood type | Reaction vessel | 1st Reagent | Sample | | 2nd Reagent |
|---|---|---|---|---|---|
| R-PHA T-PHA | 25-1-1 | | serum diluted with R-PHA buffer by ten times 25 μl | (a) | R-PHA cells dispersed in R-PHA buffer 1%    25 μl |
| | 25-1-2 | | serum diluted with R-PHA buffer by ten times 25 μl | (a) | R-PHA cells dispersed in R-PHA buffer 1%    25 μl |
| | 25-1-3 | | serum diluted with R-PHA buffer by twenty times 12.5 μl | (a) | R-PHA cells dispersed in R-PHA buffer 0.67%    37.5 μl |
| | 25-1-4 | | serum diluted with R-PHA buffer by twenty times 12.5 μl | (a) | R-PHA cells dispersed in R-PHA buffer 0.67%    37.5 μl |
| | 25-1-5 | | serum diluted with T-PHA buffer by ten times 25 μl | (b) | T-PHA cells dispersed in T-PHA buffer 1%    25 μl |
| | 25-1-6 | | serum diluted with T-PHA buffer by ten times 25 μl | (b) | T-PHA cells dispersed in T-PHA buffer 1%    25 μl |
| | 25-1-7 | | serum diluted with T-PHA buffer by twenty times 12.5 μl | (b) | T-PHA cells dispersed in T-PHA buffer 0.67%    37.5 μl |
| | 25-1-8 | | serum diluted with T-PHA buffer by twenty times 12.5 μl | (b) | T-PHA cells dispersed in T-PHA buffer 0.67%    37.5 μl |

TABLE V

| Blood type | Reaction vessel | 1st Reagent | Sample | | 2nd Reagent |
|---|---|---|---|---|---|
| R-PHA<br><br>T-PHA | 25-1-1 | | serum diluted with R-PHA buffer by ten times<br>25 µl | (a) | R-PHA cells dispersed in R-PHA buffer<br>1%    25 µl |
| | 25-1-2 | | serum diluted with R-PHA buffer by ten times<br>25 µl | (a) | R-PHA cells dispersed in R-PHA buffer<br>1%    25 µl |
| | 25-1-3 | I antibody<br><br>12.5% | serum diluted with R-PHA buffer by ten times<br>25 µl | (a) | R-PHA cells dispersed in R-PHA buffer<br>2%    12.5 µl |
| | 25-1-4 | I antibody<br><br>12.5% | serum diluted with R-PHA buffer by ten times<br>25 µl | (a) | R-PHA cells dispersed in R-PHA buffer<br>2%    12.5 µl |
| | 25-1-5 | | serum diluted with T-PHA buffer by ten times<br>25 µl | (b) | T-PHA cells dispersed in T-PHA buffer<br>1%    25 µl |
| | 25-1-6 | | serum diluted with T-PHA buffer by ten times<br>25 µl | (b) | T-PHA cells dispersed in T-PHA buffer<br>1%    25 µl |
| | 25-1-7 | absorbing reagent<br><br>12.5% | serum diluted with T-PHA buffer by ten times<br>25 µl | (b) | T-PHA cells dispersed in T-PHA buffer<br>2%    12.5 µl |
| | 25-1-8 | absorbing reagent<br><br>12.5% | serum diluted with T-PHA buffer by ten times<br>25 µl | (b) | T-PHA cells dispersed in T-PHA buffer<br>2%    12.5 µl |

In the above Tables II to V, (a) denotes the nozzles 23-1, 23-2 (52-1, 52-2) and (b) represents the nozzles 23-3, 23-4 (52-3, 52-4).

In order to effect the combinations of test items shown in the last two Tables IV and V, it is necessary to deliver only the serum sample in the sample tube 18-1-1 into the sample tubes 18-1-2 and 18-1-3 by means of the nozzles 22-1 and 22-2 under the control of the microswitch 347 shown in Fig. 12.

Now numerical examples of the timings, operation periods and operation speeds of various parts of the analyzing apparatus explained above will be described. It should be noted that these values are only denoted as exemplary ones and may be modified in various manners.

A period of operations in which the microplate is processed as a unit is about 180 seconds. That is, the periods of the advance of the sample rack 16 in the sampler 15, the supply of the microplate into the entrance of the first reaction line passage 24-1, the supply of the microplate from the exit of the third reaction line passage 24-3 into the container 40-1 and the transportation of the microplate between the reaction line passages are all about 180 seconds. A period of operations in which the sample tube column and the reaction vessel column are processed as a unit is about 15 seconds. Therefore, the period of feeding the sample rack 17 and the microplate 26 at the sample diluting position C, sample delivery position D, first and second reagent delivery positions E and G, pattern detecting position H, photographing position I and inspecting position J are all about fifteen seconds. The travelling speed of the rack transporting belts 20-1, 20-2 constituting the sample guide line 19 is 355 mm/5 seconds≅71 mm/sec. the travelling speed of the belts 28-1, 28-2 in the first reaction line passage 24-1 is 150 mm/5 seconds=30 mm/sec, the travelling speed of the belts 29-1, 29-2 in the second passage 24-2 is 14 mm/6 seconds≅2.3 mm/sec, and the travelling speed of the belts 30-1, 30-2 in the third passage 24-3 is 125 mm/6 seconds≅21 mm/sec. The belts 20-1, 20-2, 28-1, 28-2 and 30-1, 30-2 are driven intermittently for six seconds in the period of fifteen seconds. However, it should be noted that the rack 17 and microplate 26 are not moved for all this period of six seconds. The rack 17 travels from the entrance of the sample guide line 19 to the diluting position C within about five seconds. Then the sample tubes 18-1-1 to 18-1-3 in the first column are indexed at the position C and the rack is advanced by one pitch at the period of fifteen seconds. Since the rack 17 has twelve columns of sample tubes, after 15×12 seconds=3 minutes all the sample tubes have passed through the diluting position C. Then the rack 17 is fed to the sample delivery position D within two seconds during the movement of the belts 20-1, 20-2 for six seconds. At this position successive samples are delivered into the reaction vessels in the microplate at the period of fifteen seconds. After all of the samples in the rack 17 have been delivered, the rack arrives at the rack container 33 after two periods and is settled in the container. The microplate 26 is fed on the first reaction line passage 24-1 in the same manner. It takes for the microplate to advance from the entrance to E, from E to D, from D to G and from G to the stopper of the microplate transporting unit 35, five, two, two and seven seconds, respectively. Therefore, it needs two periods for the microplate to travel from the position G to the transporting unit 35. Then the microplate remains at the microplate transporting unit 35 for about three minutes and then is lifted above the belts 28-1, 28-2 by the plate 35-1 when the reaction vessels of twelfth column in a next coming microplate have arrived at the second reagent delivery position G. The lift plate 35-1 is moved upward, downward, rightward or leftward for three seconds within the period of fifteen seconds. In the second reaction line passage 24-2, the microplate 26 is fed on the belts 29-1, 29-2 at the very slow speed. On this passage 24-2, several microplates are aligned at an interval of 18 mm. Since the length of the microplate 26 is 140 mm and the length of the whole microplate including the cassette 27 is 150 mm, the microplate advances by 168 mm (150 mm+18 mm) within twelve periods. The second microplate transporting unit 36 operates in the period during which the delivery operation is effected for the reaction vessels in the first column. After the microplate has been placed on the belts 30-1, 30-2 of the third reaction line passage 24-3, the belts 30-1, 30-2 are not driven for three minutes. After that, the microplate is fed into the pattern detecting position H for two periods. When the patterns of the reaction vessels in the first column of the microplate have been detected, a next microplate is supplied onto the third reaction line passage 24-3. The microplate is fed from the pattern detecting position H to the photographing position I for two periods. When the reaction vessels in the third column are indexed at the position I, a shutter of the camera is released to take a photograph of all the patterns in the microplate. Then during next two periods the microplate is fed to the inspecting position J. Finally the microplate is settled in the container 40-1.

As explained above, since the various portions are controlled at the period of fifteen seconds, the control circuit can be made simpler. Further by changing the period into, for instance thirty seconds, the reaction time can be simply increased to sixty minutes. In this case the actually operating period of six seconds need not be changed, but only the stationary time will be increased.

Not only the blood types such as ABO-type with natural antibodies having strong agglutination, but also the blood type such as Rh-type with incomplete antibodies showing very weak agglutination can be detected accurately. Further, various kinds of antigens and antibodies in the sample serums can be equally detected in a very accurate manner. Moreover, the analyzing apparatus can be made very small in size and simple in construction. Particularly, when the microplate is fed along the reaction line passages arranged in a zig-zag form, the apparatus can be made even smaller, whereas the reaction line can still be sufficiently long. Moreover, each sample can be delivered into a plurality of reaction vessels and the analysis can be effected by judging collectively a plurality of detection results and thus, the analysis can be carried out very accurately and reliably.

The present invention is not limited to the embodiments mentioned above, but may be modified in various manners. For instance, the number of the reaction line passages is not restricted to three, but more than three passages may be provided. In this case, the number of passages is preferably set to an odd number, otherwise at the delivery positions and the detecting positions, the microplate is fed in opposite directions and thus, the reaction time for all samples could not be made the same. Further a plurality of reaction lines may be arranged three-dimensionally. For instance, the second and third passages may be arranged in a plane situated below the first passage. Moreover, in the above embodiment, the agglutination patterns formed on the reaction vessels in the same column are simultaneously detected by means of a plurality of the light receiving elements. It should be noted that the agglutination patterns of a plurality of reaction vessels may be successively detected by scanning them with a single detector which is movable in a width direction of the microplate. Further, in the above embodiments, the bottom surface of each reaction vessel formed in the microplate is made conical, but may be formed in various shapes such as polygonal pyramid, gable roof and hip roof.

**Claims**

1. An analyzing method for agglutinating reaction comprising

supplying carriers (26) successively into an entrance of a reaction line (24), each carrier (26) having therein a plurality of reaction vessels (25) each having an inclined bottom surface;

delivering given amounts of sample liquids into the reaction vessels (25) of each carrier (26), while the carrier (26) is fed along the reaction line (24);

delivering given amounts of reagents into the reaction vessels (25) of each carrier (26), while the carrier (26) is fed along the reaction line (24);

feeding each carrier (26) along the reaction line (24) while the reactions take place in test liquids in the reaction vessels (25), said test liquids being mixtures of samples and reagents and containing particles;

detecting photoelectrically agglutination formed in the reaction vessels (25) to effect analyses in accordance with existence and non-existence of agglutination; and

discharging each carrier (26) from an exit of the reaction line (24), characterized in that said feeding comprises feeding each carrier (26) in a steady manner and thus without subjecting said test liquids in its reaction vessels (25) to sharp shocks, said detecting comprises detecting photoelectrically agglutination patterns formed on the bottom surfaces of the reaction vessels (25) by the particles, and the bottom surface of each reaction vessel (25) is inclined to the horizontal in such a manner and is provided with a plurality of such steps (26-5) that the motion of the agglutinated particles down the bottom surface is inhibited whilst rolling of the non-agglutinated particles down the bottom surface is less inhibited.

2. A method according to claim 1, wherein each sample liquid is delivered into a plurality of reaction vessels (25) in a carrier (26) to form a plurality of agglutination patterns and analyzing results are derived by judging collectively all detection results of these plurality of agglutination patterns.

3. A method according to claim 1 or 2, wherein said carriers (26) are fed along the reaction line (24) comprising at least three reaction line passes (24-1, 24-2, 24-3) arranged in a zig-zag manner.

4. A method according to claim 3, wherein the sample delivering and the reagent delivering are effected while each carrier (26) is fed along the first reaction line passage (24-1) and the agglutination pattern detecting is carried out while the carrier (26) is fed along the last reaction line passage (24-3).

5. A method according to claim 4, wherein in the first and last reaction line passages (24-1, 24-3) the carriers (26) are fed intermittently and in the intermediate reaction line passage(s) (24-2) the carriers (26) are fed continuously.

6. A method according to claim 3, 4, or 5, wherein said sample liquids are contained in sample vessels (18) in a sample rack (17) and the sample rack (17) is fed along a sample line (19) arranged in parallel with the first reaction line .passage (24-1) in an opposite direction to a direction in which the carriers (26) are fed along said first reaction line passage (24-1).

7. A method according to claim 6, wherein the .sample rack (17) and the carriers (26) are indexed on the sample line (19) and the first reaction line passage (24-1), respectively at first and second reagent delivery positions (E, G).

8. A method according to claim 7, wherein said sample delivering is effected at a sample delivery position (D) between said first and second reagent delivery positions (E, G).

9. A method according to claim 1, wherein the agglutination pattern detecting is effected simultaneously for all of the reaction vessels (25-1 to 25-8) aligned in a column in each carrier (26).

10. A method according to any preceding claim, wherein the method further comprises taking a photograph of all of the agglutination patterns formed on the bottom surfaces of the reaction vessels (25) in a single carrier (26).

11. A method according to claim 10, wherein the photographing is effected after the agglutination pattern detecting.

12. A method according to any preceding claim, wherein the carriers (26) discharged from the exit of the reaction line (24) are stacked one upon another.

13. A method for analyzing blood samples according to any preceding claim, wherein each blood sample is separated by a centrifuge into a blood cell sample and a serum sample, the blood cell and serum samples are diluted to form a blood cell suspension and a serum dilution, respectively, and the blood cell suspension and serum dilution are delivered into respective reaction vessels (25) in the same carrier (26).

14. A method according to any preceding claim, wherein each carrier (26) is formed by a microplate (26) and a cassette (27) for removably containing the microplate (26).

15. A method according to any one of claims 1 to 13, wherein each carrier (26) is formed by two microplate halves (26A, 26B) and a cassette (27) for removably containing the microplate halves (26A, 26B).

16. A method according to claim 15, wherein said microplate halves (26A, 26B) have reaction vessels (25A, 25B) having different bottom surface configuration and/or size.

17. An analyzing apparatus for agglutinating reaction comprising
means (28, 29, 30) for feeding carriers (26) each having therein a plurality of reaction vessels (25) having inclined bottom surfaces, along a reaction line (24);
means (17, 23) for delivering given amounts of samples successively into the reaction vessels (25), while the carriers (26) are fed along the reaction line (24);
means (31, 34) for delivering given amounts of reagents into the reaction vessels (25), while the carriers (26) are fed along the reaction line (24);
means (37) for detecting photoelectrically agglutination in the reaction vessels (25) to produce detection signals; and
means (38) for receiving the detection signals to effect an analysis through existence and non-existence of the agglutination;
characterized in that the feeding means (28, 29, 30) comprises means (29) for feeding said carriers (26), when said carriers (26) contain said samples and said reagents, along said reaction line (24-2) in a steady manner and thus without subjecting said carriers (26) to sharp shocks, in that the detecting means (37) serves to detect agglutination patterns of particles on the inclined bottom surfaces of the reaction vessels (25), and in that the bottom surface of each reaction vessel (25) is inclined to the horizontal in such a manner and is provided with a plurality of such steps (26-5) that the motion of the agglutinated particles down the bottom surface is inhibited whilst rolling of the non-agglutinated particles down the bottom surface is less inhibited.

18. An apparatus according to claim 17, wherein the sample delivering means (17, 23) delivers each sample liquid into a plurality of reaction vessels (25) formed in a carrier (26) to form a plurality of agglutination patterns, said detecting means (37) produces a plurality of detection signals representing these plurality of agglutination patterns, and analysis is effected by judging collectively said detection signals.

19. An apparatus according to claim 17 or 18, wherein said reaction line (24) comprises at least three reaction line passages (24-1, 24-2, 24-3) arranged in a zig-zag manner, and said feeding means (28, 29, 30) comprises at least two transporting devices (35, 36) for transporting the carrier (26) between the reaction line passages (24-1, 24-2, 24-3).

20. An apparatus according to claim 19, wherein the sample delivering means (17, 23) and the reagent delivering means (31, 34) are arranged along the first reaction line passage (24-1) and said detecting means (37) is arranged on the last reaction line passage (24-3).

21. An apparatus according to claim 20, wherein said feeding means (28, 29, 30) feeds the carriers (26) along the first and last reaction line passages (24-1, 24-3) intermittently and along the intermediate reaction line passage(s) (24-2) continuously.

22. An apparatus according to claim 19, 20, or 21 wherein said sample delivering means (17, 23) comprises sample racks (17) each including a plurality of sample vessels (18) in which the samples are contained, a mechanism (20) for feeding the sample racks (17) along a sample line (19) arranged in parallel with the first reaction line passage (24-1) in an opposite direction to a direction in which the carriers (26) are fed along said first reaction line passage (24-1), and a delivery arrangement (23) for aspirating given amounts of the samples and discharging the aspirated samples into the reaction vessels (25).

23. An apparatus according to claim 22, wherein said delivery arrangement (23) is provided at a sample delivery position (D) at which each sample rack (17) is indexed on the sample line (19) and said feeding means (28, 29, 30) has mechanisms (21-3, 21-5) for indexing each carrier (26) at first and second reagent delivery positions (E, G) along the first reaction line passage (24-1).

24. An apparatus according to claim 23, wherein said delivery arrangement (23) is arranged between said first and second reagent delivery positions (E, G) on the first reaction line passage (24-1).

25. An apparatus according to any one of claims 17 to 24, wherein said detecting means (37) comprises an illumination device (100) for illuminating a plurality of reaction vessels (25) aligned in a column in a carrier (26), an optical system (103) for forming images of the agglutination patterns formed in said plurality of reaction vessels (25) and a plurality of light receiving elements (104-1 to 104-8) for receiving said images respectively.

26. An apparatus according to any one of claims 17 to 25, wherein the apparatus further comprises a camera for taking a photograph of all the agglutination patterns formed on the bottom surfaces of reaction vessels (25) in a single carrier (26).

27. An apparatus according to claim 26, wherein said camera is arranged downstream of the detecting means (37).

28. An apparatus according to any one of claims 17 to 27, and further comprising means (40) for receiving the carriers (26) discharged from the exit of the reaction line (24) and stacking them one upon another.

29. An apparatus for analyzing blood samples according to any one of claims 17 to 28, wherein each blood sample is separated by a centrifuge into a blood cell sample and a serum sample, and said apparatus further comprises a sample diluting unit (300) for diluting the blood cell and serum samples to form a blood cell suspension and a serum dilution, respectively and at least two sample delivering devices (23-1 to 23-4) for delivering the blood cell suspension and serum dilution into respective reaction vessels (25) in the same carrier (26).

30. An apparatus according to any one of claims 17 to 29, wherein each carrier (26) comprises a microplate (26) and a cassette (27) for removably containing the microplate (26).

31. An apparatus according to any one of claims 17 to 29, wherein each carrier (26) comprises two microplate halves (26A, 26B) and a cassette (27) for removably containing the microplate halves (26A, 26B).

32. An apparatus according to claim 31, wherein said microplate halves (26A, 26B) have reaction vessels (25) having different bottom surface configuration and/or size.

33. An apparatus according to any one of claims 19 to 24, or any one of claims 25 to 32 as appended to claim 19, wherein said feeding means (28, 29, 30) in each of said reaction line passages (24-1, 24-2, 24-3) comprises a pair of endless belts (28-1, 28-2, 29-1, etc.) on which said carriers (26) are placed.

34. An apparatus according to claim 33, wherein each transporting device (35, 36) comprises a plate member (35-1, 36-1) on which each carrier (26) is placed and a mechanism (457 to 465) for moving said plate member (35-1, 36-1) up and down between said pair of belts (28-1, 28-2, 29-1, etc.) and for moving horizontally the plate member (35-1, 36-1) between the reaction line passages (24-1, 24-2, 24-3).

35. An apparatus according to any one of claims 17 to 22, wherein said feeding means (28, 29, 30) comprises means (21-3, 21-4, 21-5) for indexing the carriers (26) at the sample and reagent delivering means (23, 31, 34).

36. An apparatus according to claim 35, wherein each indexing means (21-3, 21-4, 21-5) comprises a pair of claws (21-12, 21-13) which are selectively engaged with indexing members (27-7) formed on at least one side wall (27) of each carrier (26).

37. An apparatus according to claim 36, wherein said indexing members (27-7) are composed of projections (27-7).

38. An apparatus according to claim 36 or 37 and claim 30, 31, or 32, wherein said indexing members (27-7) are formed on at least one side wall (27) of each cassette (37).

**Patentansprüche**

1. Analyseverfahren für eine Agglutinationsreaktion, mit folgenden Verfahrensschritten:

— sukzessiver Zuführung von Trägern (26) in den Einlaß einer Reaktionslinie (24), wobei jeder Träger (26) mehrere Reaktionsgefäße (25) aufweist, von denen jedes eine geneigte Bodenfläche aufweist,
— Zugeben von vorgegebenen Mengen an Probenflüssigkeiten in die Reaktionsgefäße (25) eines jeden Trägers (26), während der Träger (26) entlang der Reaktionslinie (24) bewegt wird,
— Zugeben von vorgegebenen Mengen an Reagenzien in die Reaktionsgefäße (25) eines jeden Trägers (26), während der Träger (26) entlang der Reaktionslinie (24) bewegt wird,
— Bewegen eines jeden Trägers (26) entlang der Reaktionslinie (24), während die Reaktionen in Testflüssigkeiten in den Reaktionsgefäßen (25) ablaufen, wobei die Testflüssigkeiten Mischungen aus Proben und Reagenzien sind und Partikel enthalten,
— fotoelektrischer Erkennung einer in den Reaktionsgefäßen (25) gebildeten Agglutination zur Durchführung von Analysen im Hinblick auf die Existenz oder Nichtexistenz von Agglutinationen und
— Ausgeben eines jeden Trägers (26) aus einem Auslaß der Reaktionslinie (24),

dadurch gekennzeichnet,

— daß das Bewegen eines jeden Trägers (26) durch stetiges Bewegen erfolgt, derart, daß die Testflüssigkeiten in ihren Reaktionsgefäßen (25) keinen starken Stößen ausgesetzt werden,
— daß die Erkennung die fotoelektrische Erkennung von Agglutinations-Mustern umfaßt, die durch die Partikel auf den Bodenflächen der Reaktionsgefäße (25) gebildet werden, und
— daß die Bodenfläche eines jeden Reaktionsgefäßes (25) derart zur Horizontalen geneigt und mit einer Vielzahl von solchen Stufen (26-5) versehen ist, daß die Abwärtsbewegung der Agglutinations-Partikel auf der Bodenfläche behindert wird, während das Herabrollen der Nichtagglutinations-Partikel auf der Bodenfläche weniger stark behindert wird.

2. Verfahren nach Anspruch 1, bei dem jede Probenflüssigkeit zur Bildung mehrerer Agglutinations-Muster in mehrere Reaktionsgefäße (25) in einem Träger (26) gegeben wird und die Analysenresultate durch kollektive Beurteilung aller Erkennungsresultate dieser Vielzahl von Agglutinations-Mustern gewonnen werden.

3. Verfahren nach Anspruch 1 oder 2, bei dem die Träger (26) entlang der mindestens drei zickzackförmig angeordnete Reaktionslinienabschnitte (24-1, 24-2, 24-3) aufweisenden Reaktionslinie (24) bewegt werden.

4. Verfahren nach Anspruch 3, bei dem das Zugeben der Proben und das Zugeben der Reagenzien ausgeführt wird, während jeder Träger (26) entlang des ersten Reaktionslinienabschnittes (24-1) bewegt wird, und die Agglutinations-Mustererkennung ausgeführt wird, während der Träger (26) entlang des letzten Reaktionslinienabschnittes (24-3) bewegt wird.

5. Verfahren nach Anspruch 4, bei dem die Träger (26) im ersten und letzten Reaktionslinienabschnitt (24-1, 24-3) intermittierend bewegt werden, und die Träger (26) in dem bzw. den dazwischenliegenden Reaktionslinienabschnitt(-en) (24-2) gleichmäßig bewegt werden.

6. Verfahren nach Anspruch 3, 4 oder 5, bei dem die Probenflüssigkeiten in Probengefäßen (18) in einem Probengestell (17) enthalten sind und das Probengestell (17) entlang einer parallel zum ersten Reaktionslinienabschnitt (24-1) angeordneten Probenlinie (19) in entgegengesetzter Richtung zu derjenigen Richtung bewegt wird, in der die Träger (26) entlang des ersten Reaktionslinienabschnittes (24-1) bewegt werden.

7. Verfahren nach Anspruch 6, bei dem das Probengestell (17) und die Träger (26) auf der Probenlinie (19) bzw. dem ersten Reaktionslinienabschnitt (24-1) an ersten und zweiten Reagenzienzugabepositionen (E, G) indiziert werden.

8. Verfahren nach Anspruch 7, bei dem die Probenzugabe an einer Probenzugabeposition (D) zwischen den ersten und zweiten Reagenzienzugabepositionen (E, G) ausgeführt wird.

9. Verfahren nach Anspruch 1, bei dem die Agglutinations-Mustererkennung gleichzeitig für alle in einer Spalte eines jeden Trägers (26) ausgerichteten Reaktionsgefäße (25-1 bis 25-8) ausgeführt wird.

10. Verfahren nach einem der vorhergehenden Ansprüche, bei dem das Verfahren zusätzlich die fotografische Aufnahme aller Agglutinations-Muster umfaßt, die auf den Bodenflächen der Reaktionsgefäße (25) in einem einzigen Träger (26) erzeugt worden sind.

11. Verfahren nach Anspruch 10, bei dem das Fotografieren nach der Agglutinations-Mustererkennung ausgeführt wird.

12. Verfahren nach einem der vorhergehenden Ansprüche, bei dem die aus dem Auslaß der Reaktionslinie (24) ausgegebenen Träger (26) übereinandergestapelt werden.

13. Verfahren zum Analysieren von Blutproben nach einem der vorhergehenden Ansprüche, bei dem jede Blutprobe durch eine Zentrifuge in eine Blutzellenprobe und eine Serumprobe getrennt wird, die Blutzellenproben und die Serumproben jeweils zur Bildung einer Blutzellensuspension und einer Serumdilution verdünnt werden und die Blutzellensuspension und die Serumdilution in entsprechende Reaktionsgefäße (25) in demselben Träger (26) gegeben werden.

14. Verfahren nach einem der vorhergehenden Ansprüche, bei dem jeder Träger (26) durch eine Mikroplatte (26) und eine Kassette (27) zum entnehmbaren Aufnehmen der Mikroplatte (26) gebildet wird.

15. Verfahren nach einem der Ansprüche 1 bis 13, bei dem jeder Träger (26) durch zwei Mikroplattenhälften (26A, 26B) und eine Kassette (27) zum entnehmbaren Aufnehmen der Mikroplattenhälften (26A, 26B) gebildet wird.

16. Verfahren nach Anspruch 15, bei dem die Mikroplattenhälften (26A, 26B) Reaktionsgefäße (25A, 25B) mit unterschiedlicher Bodenflächenkonfiguration und/oder Größe aufweisen.

17. Analysengerät für Agglutinations-Reaktionen mit

— Mitteln (28, 29, 30) zum Bewegen von Trägern (26) entlang einer Reaktionslinie (24B), wobei jeder Träger (26) im Innern mehrere Reaktionsgefäße (25) mit geneigten Bodenflächen aufweist,

— Mitteln (17, 23) zum sukzessiven Zugeben von vorgegebenen Mengen an Proben in die Reaktionsgefäße (25), während die Träger (26) entlang der Reaktionslinie (24) bewegt werden,

— Mitteln (31, 34) zum Zugeben von vorgegebenen Mengen an Reagenzien in die Reaktionsgefäße (25), während die Träger (26) entlang der Reaktionslinie (24) bewegt werden,

— Mitteln (37) zum fotoelektrischen Erkennen von Agglutinationen in den Reaktionsgefäßen (25) zur Erzeugung von Detektionssignalen, und

— Mitteln (38) zum Empfangen der Detektionssignale, um durch Existenz oder Nichtexistenz der Agglutination eine Analyse auszuführen,

dadurch gekennzeichnet,

— daß die Bewegungsmittel (28, 29, 30) Mittel (29) zum stetigen Bewegen der Träger (26), wenn diese Träger (26) die Proben und die Reagenzien enthalten, entlang der Reaktionslinie (24-2) und daher ohne Ausübung scharfer Stöße auf die Träger (26) aufweisen,

— daß die Erkennungsmittel (37) dazu dienen, Agglutinations-Muster der Partikel auf den schrägen Bodenflächen der Reaktionsgefäße (25) zur erkennen und

— daß die Bodenfläche eines jeden Reaktionsgefäßes (25) zur Horizontalen derart geneigt und mit mehreren derartigen Stufen (26-5) versehen ist, daß die Abwärtsbewegung der Agglutinations-Partikel auf der Bodenfläche behindert wird, während das Herabrollen der Nichtagglutinations-Partikel auf der Bodenfläche weniger stark behindert wird.

18. Gerät nach Anspruch 17, bei dem die Probenzugabemittel (17, 23) zur Erzeugung mehrerer Agglutinations-Muster jede Probenflüssigkeit in mehrere in einem Träger (26) gebildete Reaktionsgefäße (25) geben, die Erkennungsmittel (37) mehrere diese Vielzahl von Agglutinations-Muster repräsentierende Detektionssignale erzeugen und die Analyse durch kollektive Beurteilung der Detektionssignale ausgeführt wird.

19. Gerät nach Anspruch 17 oder 18, bei dem die Reaktionslinie (24) mindestens drei zickzackförmig angeordnete Reaktionslinienabschnitte (24-1, 24-2, 24-3) umfaßt und die Bewegungsmittel (28, 29, 30) mindestens zwei Transporteinrichtungen (35, 36) zum Transportieren der Träger (26) zwischen den Reaktionslinienabschnitten (24-1, 24-2, 24-3) umfassen.

20. Gerät nach Anspruch 19, bei dem die Probenzugabemittel (17, 23) und die Reagenzienzugabevorrichtung (31, 34) entlang des ersten Reaktionslinienabschnittes (24-1) angeordnet sind und die Erkennungsmittel (37) an dem letzten Reaktionslinienabschnitt (24-3) angeordnet sind.

21. Gerät nach Anspruch 20, bei dem die Bewegungsmittel (28, 29, 30) die Träger (26) entlang des ersten und des letzten Reaktionslinienabschnittes (24-1, 24-3) intermittierend und entlang des bzw. den dazwischenliegenden Reak-tionslinienabschnittes bzw. Reaktionslinienab-schnitten (24-2) gleichmäßig bewegen.

22. Gerät nach Anspruch 19, 20 oder 21, bei dem die Probenzugabemittel (17, 23) Probengestelle (17), von denen jedes mehrere die Proben enthaltende Probengefäße (18) aufweist, einen Mechanismus (20) zum Bewegen der Probengestelle (17) entlang einer parallel zum ersten Reaktionslinienabschnitt (24-1) angeordneten Probenlinie (19) in einer Richtung, die entgegengesetzt zu derjenigen Richtung ist, in der die Träger (26) entlang des ersten Reaktionslinienabschnittes (24-1) bewegt werden, und eine Zugabe-Anordnung (23) zum Absaugen von vorgegebenen Mengen an Proben und zum Zugeben der abgesaugten Proben in die Reaktionsgefäße (25) aufweist.

23. Gerät nach Anspruch 22, bei dem die Zugabe-Anordnung (23) an einer Probenzugabeposition (D) angeordnet ist, an der jedes Probengestell (17) auf der Probenlinie (19) indiziert wird, und die Bewegungsmittel (28, 29, 30) einen Mechanismus (21-3, 21-5) zum Indizieren eines jeden Trägers (26) an der ersten und der zweiten Reagenzienzugabeposition (E, G) entlang des ersten Reaktionslinienabschnittes (24-1) aufweisen.

24. Gerät nach Anspruch 23, bei dem die Zugabe-Anordnung (23) zwischen der ersten und der zweiten Reagenzienzugabeposition (E, G) an dem ersten Reaktionslinienabschnitt (24-1) angeordnet ist.

25. Gerät nach einem der Ansprüche 17 bis 24, bei dem die Erkennungsmittel (37) eine Beleuchtungseinrichtung (100) zum Beleuchten von mehreren in einer Spalte in einem Träger (26) ausrichtete Reaktionsgefäßen (25), ein optisches System (103) zum Erzeugen von Bildern der Agglutinations-Mustern, die in den mehreren Reaktionsgefäßen (25) gebildet sind und mehrere lichtempfangende Elemente (104-1 bis 104-8) aufweisen, die die Bilder jeweils empfangen.

26. Gerät nach einem der Ansprüche 17 bis 25, bei dem ferner eine Kamera zur fotografischen Aufnahme aller auf den Bodenflächen der Reaktionsgefäße (25) in einem einzigen Träger (26) gebildeten Agglutinations-Muster vorgesehen ist.

27. Gerät nach Anspruch 26, bei dem die Kamera stromabwärts von der Erkennungsmittel (37) angeordnet ist.

28. Gerät nach einem der Ansprüche 17 bis 27, das darüber hinaus mit Mittel (40) zum Aufnehmen der aus dem Auslaß der Reaktionslinie (24) ausgegebenen Träger (26) und zum Übereinanderstapeln derselben versehen ist.

29. Gerät zum Analysieren von Blutproben nach einem der Ansprüche 17 bsi 28, bei dem jede Blutprobe durch eine Zentrifuge in eine Blutzellenprobe und eine Serumprobe aufgeteilt wird, und das Gerät ferner eine Proben-Verdünnungseinheit (300) zum Verdünnen der Blutzellen- und Serumproben aufweist, um jeweils eine Blutzellensuspension und eine Serumdilution zu erzeugen, und mindestens zwei Probenzugabemittel (23-1 bis 23-4) zum Zugeben der Blutzellensuspension und der Serumdilution in entsprechende Reaktionsgefäße (25) in demselben Träger (26) aufweist.

30. Gerät nach einem der Ansprüche 17 bis 29, bei dem jeder Träger (26) eine Mikroplatte (26) und eine Kassette (27) zum entnehmbaren Aufnehmen der Mikroplatte (26) aufweist.

31. Gerät nach einem der Ansprüche 17 bis 29, bei dem jeder Träger (26) zwei Mikroplattenhälften (26A, 26B) und eine Kassette (27) zum entnehmbaren Aufnehmen der Mikroplattenhälften (26A, 26B) aufweist.

32. Gerät nach Anspruch 31, bei dem jede Mikroplattenhälfte (26A, 26B) Reaktionsgefäße (25) mit unterschiedlicher Bodenflächenkonfiguration und/oder Größe aufweist.

33. Gerät nach einem der Ansprüche 19 bis 24 oder einem der auf Anspruch 19 rückbezogenen Ansprüche 25 bis 32, bei dem die Bewegungsmittel (28, 29, 30) in jedem der Reaktionslinienabschnitte (24-1, 24-2, 24-3) ein Paar endlose Bänder (28-1, 28-2, 29-1, usw.) aufweisen, auf denen die Träger (26) plaziert sind.

34. Gerät nach Anspruch 33, bei dem jede Transporteinrichtung (35, 36) ein Plattenelement (35-1, 36-1), auf dem jeder Träger (26) angeordnet ist, und einen Mechanismus (457 bis 465) zum Auf- und Abbewegen der Plattenelemente (35-1, 36-1) zwischen dem Paar von Bändern (28-1, 28-1, 29-1, usw.) und zum horizontalen Bewegen des Plattenelements (35-1, 36-1) zwischen den Reaktionslinienabschnitten (24-1, 24-2, 24-3) aufweist.

35. Gerät nach einem der Ansprüche 17 bis 22, bei dem die Bewegungsmittel (28, 29, 30) Mittel (21-3, 21-4, 21-5) zum Indizieren der Träger (26) an den Proben- und Reagenzienzugabevorrichtungen (23, 31, 34) aufweisen.

36. Gerät nach Anspruch 35, bei dem jedes Indizierungsmittel (21-3, 21-4, 21-5) ein Paar Klauen (21-12, 21-13) aufweist, die wahlweise an auf mindestens einer Seitenwand (27) eines jeden Trägers (26) gebildeten Indexelementen (21-7) angreifen.

37. Gerät nach Anspruch 36, bei dem die Indexelemente (27-7) aus Vorsprüngen (27-7) bestehen.

38. Gerät nach Anspruch 36 oder 37 und Anspruch 30, 31 oder 32, bei dem die Indexelemente (27-7) auf mindestens einer Seitenwand (27) einer jeden Kassette (27) ausgebildet sind.

### Revendications

1. Procédé d'analyse pour réaction d'agglutination comprenant:

l'alimentation successive de supports (26) dans une entrée d'une ligne de réaction (24), chaque support (26) comportant une pluralité de vases de réaction (25) ayant chacun une surface de fond en plan incliné;

l'alimentation de quantités données de liquides échantillons dans les vases de réaction (25) de chaque support (26), pendant que le support (26) est avancé le long de la ligne de réaction (24);

l'alimentation de quantités données de réactifs dans les vases de réaction (25) de chaque support (26), pendant que le support (26) est avancé le long de la ligne de réaction (24);

l'avance de chaque support (26) le long de la ligne de réaction (24) pendant que les réactions ont lieu dans les liquides tests dans les vases de réaction (25), lesdits liquides tests étant des mélanges d'échantillons et de réactifs, et contenant des particules;

détection photo-électrique de l'agglutination formée dans les vases de réaction (25) pour l'exécution d'analyses en fonction de l'existence et de la non-existence d'agglutination; et

déchargement de chaque support (26) à partir d'une sortie de la ligne de réaction (24),

ledit procédé étant caractérisé en ce que ladite avance prévoit l'avance de chaque support (26) d'une manière stable et par conséquent sans soumettre à des chocs brusques lesdits liquides tests dans leurs vases de réaction (25), ladite détection comprenant la détection photo-électrique de schémas d'agglutination formés sur les surfaces de fond des vases de réaction (25) par les particules, et la surface de fond de chaque vase de réaction (25) est inclinée par rapport à l'horizontale en étant pourvue d'une pluralité d'échelons (26-5) de manière que le mouvement en direction du bas des particules agglutinées le long de la surface de fond soit freiné, tandis que le roulement en direction du bas des particules non agglutinées le long de la surface de fond est moins gêné.

2. Procédé selon la revendication 1, caractérisé en ce que chaque liquide échantillon est alimenté dans une pluralité de vases de réaction (25) dans un support (26) de façon à former une pluralité de schémas d'agglutination, les résultats d'analyse étant dérivés en jugeant collectivement de tous les résultats de détection de cette pluralité de schémas d'agglutination.

3. Procédé selon la revendication 1 ou 2, caractérisé en ce que lesdits supports (26) sont avancés le long de la ligne de réaction (24) comprenant au minimum trois passages de ligne de réaction (24-1, 24-2, 24-3) disposés de manière en zig-zag.

4. Procédé selon la revendication 3, caractérisé en ce que l'alimentation de l'échantillon et l'alimentation du réactif sont effectuées pendant que chaque support (26) est avancé le long du premier passage (24-1) de la ligne de réaction, et la détection du schéma d'agglutination est effectuée pendant que le support (26) est avancé le long du dernier passage (24-3) de la ligne de réaction.

5. Procédé selon la revendication 4, caractérisé en ce que, dans le premier et le dernier passages (24-1, 24-3) de la ligne de réaction, les supports (26) sont avancés par intermittence, et dans le passage intermédiaire ou les passages intermédiaires (24-2) de la ligne de réaction, les supports (26) sont avancés en continu.

6. Procédé selon la revendication 3, 4 ou 5, caractérisé en ce que lesdites échantillons liquides sont contenus dans des vases à échantillons (18) dans un râtelier à échantillons (17), et le râtelier à échantillons (17) est avancé le long d'une ligne d'échantillonnage (19) disposée en parallèle avec le premier passage (24-1) de la ligne de réaction et dans un sens opposé au sens dans lequel les supports (26) sont avancés le long dudit premier passage (24-1) de la ligne de réaction.

7. Procédé selon la revendication 6, caractérisé en ce que le râtelier à échantillons (17) et les supports (26) sont inedexés sur la ligne d'échantillonnage (19) et le premier passage (24-1) de la ligne de réaction, respectivement à une première et une deuxième position d'alimentation de réactif (E, G).

8. Procédé selon la revendication 7, caractérisé en ce que la dite alimentation d'échantillon est effectuée à uen position d'alimentation d'échantillon (D) entre ladite première et ladite deuxième position d'alimentation de réactif (E, G).

9. Procédé selon la revendication 1, caractérisé en ce que la détection des schémas d'agglutination est effectuée simultanément pour tous les vases de réaction (25-1 à 25-8) alignés dans une colonne dans chaque support (26).

10. Procédé selon une quelconque des revendications précédentes, caractérisé en ce qu'il comprend la prise d'une photographie de tous les schémas d'agglutination formés sur les surfaces de fond des vases de réaction (25) dans un support unitaire (26).

11. Procédé selon la revendication 10, caractérisé en ce que la photographie est effectuée après la détection des schémas d'agglutination.

12. Procédé selon une quelconque des revendications précédentes, caractérisé en ce que les supports (26) évacués de la sortie de la ligne de réaction (24) sont empilés l'un sur l'autre.

13. Procédé pour l'analyse d'échantillons de sang selon une quelconque des revendications précédentes, caractérisé en ce que chaque échantillon de sang est séparé par un centrifuge en un échantillon de sérum, les échantillons de globules du sang et de sérum étant dilués de manière à former une suspension de globules du sang et une dilution de sérum, respectivement, la suspension de globules du sang et la dilution de sérum étant alimentées dans des vases de réaction respectifs (25) dans le même support (26).

14. Procédé selon une quelconque des revendications précédentes, caractérisé en ce que chaque support (26) est constitué par une micro-plaque (26) et une cassette (27) destinée à contenir de manière amovible la micro-plaque (26).

15. Procédé selon une quelconque des revendications 1 à 13, caractérisé en ce que chaque support (26) est constitué par deux moities de microplaque (26A, 26B) et par une cassette (27) destinée à contenir de manière amovible les moitiés de microplaque (26A, 26B).

16. Procédé selon la revendication 15, caractérisé en ce que lesdites moitiés de microplaque (26A, 26B) comportent des vases de réaction (25A, 25B) ayant une configuration et/ou une dimension de surface de fond différentes.

17. Appareil d'analyse pour réaction d'agglutination comprenannt:

des moyens (28, 29, 30) pour l'avance, le long d'une ligne de réaction (24), de supports (26) comportant chacun une pluralité de vases de réaction (25) ayant des surfaces de fond inclinées;

des moyens (17, 23) pour alimenter des quantités données d'échantillons successivement dans les vases de réaction (25) pendant que les supports (26) sont avancés le long de la ligne de réaction (24);

des moyens (31, 34) pour alimenter des quantités données de réactifs dans les vases de réaction (25), pendant que les supports (26) sont avancés le long de la ligne de réaction (24);

un moyen pour détecter photoélectriquement l'agglutination dans les vases de réaction (25) de manière à produire des signaux de détection; et

un moyen (38) pour la réception des signaux de détection de manière à effectuer une analyse de l'existence et de la non-existence de l'agglutination,

ledit appareil étant caractérisé en ce que les moyens d'avance (28, 29, 30) comprennent un moyen (29) pour l'avance desdits supports (26), lorsque lesdits supports (26) contiennent lesdits échantillons et lesdits réactifs, le long de ladite ligne de réaction (24-2) de manière stable et par conséquent sans soumettre lesdits supports (26) à des chocs brusques; en ce que le moyen de détection (37) sert à détecter les schémas d'agglutination de particules sur les surfaces de fond en plan incliné des vases de réaction (25); et en ce que la surface de fond de chaque vase de réaction (25) est en plan incliné par rapport à l'horizontale et est pourvue d'une pluralité de degrés ou échelons (26-5) de manière que le mouvement en direction du bas des particules agglutinées le long de la surface de fond soit freiné, tandis que le roulement en direction du bas des particules non agglutinées le long de la surface de fond est moins freiné.

18. Appareil selon la revendication 17, caractérisé en ce que les moyens d'alimentation (17, 23) débitent chaque liquide échantillon dans une pluralité de vases de réaction (25) formés dans un support (26), de manière à constituer une pluralité de schémas d'agglutination, ledit moyen de détection (37) produisant une pluralité de signaux de détection représentant cette pluralité de schémas d'agglutination, l'analyse étant effectuée en jugeant collectivement desdits signaux de détection.

19. Appareil selon la revendication 17 ou 18, caractérisé en ce que ladite ligne de réaction (24) comprend au moins trois passages de ligne de réaction (24-1, 24-2, 24-3) disposés d'une manière en zig-zag, et lesdits moyens d'avance (28, 29, 30) comprennent au minimum deux dispositifs de transport (35, 36) pour le transport du support (26) entre les passages de ligne de réaction (24-1, 24-2, 24-3).

20. Appareil selon la revendication 19, caractérisé en ce que les moyens d'alimentation d'échantillons (17, 23) et les moyens d'alimentation de réactif (31, 34) sont disposés le long du premier passage (24-1) de la ligne de réaction, et ledit moyen de détection (37) est disposé sur le dernier passage (24-3) de la ligne de réaction.

21. Appareil selon la revendication 20, caractérisé en ce que lesdits moyens d'alimentation (28, 29, 30) ou d'avance font avancer les supports (26) le long du premier et du dernier passage de ligne de réaction (24-1, 24-3) de manière intermittente, et font avancer ces supports en continu le long du passage intermédiaire ou des passages intermédiaires (24-2) de la ligne de réaction.

22. Appareil selon la revendication 19, 20, ou 21, caractérisé en ce que lesdits moyens d'alimentation d'échantillons (17, 23) comprennent: des râteliers à échantillons (17) incluant chacun une pluralité de vases à échantillons (18) dans lesquels les échantillons sont contenus; un mécanisme (20) pour l'avance des râteliers à échantillons (17) le long d'une ligne d'échantillonnage (19) disposée en parallèle par rapport au premier passage (24-1) de la ligne de réaction, en sens inverse au sens dans lequel les supports (26) sont avancés le long dudit premier passage (24-1) de la ligne de réaction; et un système d'alimentation (23) pour l'aspiration de quantités données des échantillons et pour la décharge des échantillons aspirés dans les vases de réaction (25).

23. Appareil selon la revendication 22, caractérisé en ce que ledit système d'alimentation (23) est prévu à une position d'alimentation d'échantillon (D) à laquelle chaque râtelier à échantillons (17) est indexé sur la ligne d'échantillonnage (19), les dits moyens d'avance (28, 29, 30)

30

comportant des mécanismes (21-3, 21-5) pour l'indexation de chaque support (26) à la première et à la deuxième position d'alimentation de réactif (E, G) le long du premier passage (24-1) de la ligne de réaction.

24. Appareil selon la revendication 23, caractérisé en ce que ledit système d'alimentation (23) est prévu entre ladite première et ladite deuxième position d'alimentation de réactif (E, G) sur le premier passage (24-1) de la ligne de réaction.

25. Appareil selon une quelconque des revendications 17 à 24, caractérisé en ce que ledit moyen de détection (37) comprend un dispositif d'éclairage (100) pour l'éclairage d'une pluralité de vases de réaction (25) alignés en une colonne dans un support (26); un système optique (103) pour la formation d'images des schémas d'agglutination constitués dans ladite pluralité de vases de réaction (25); et une pluralité d'éléments photo-récepteurs (104-1, à 104-8) pour la réception desdites images respectivement.

26. Appareil selon une quelconque des revendications 17 à 25, caractérisé en ce que l'appareil comprend en outre une caméra pour la prise d'une photographie de tous les schémas d'agglutination formés sur les surfaces de fond des vases de réaction (25) dans un support unitaire (26).

27. Appareil selon la revendication 26, caractérisé en ce que ladite caméra est disposée en aval du moyen de détection (37).

28. Appareil selon une quelconque des revendications 17 à 27, caractérisé en ce qu'il comprend en outre un moyen (40) pour la réception des supports (26) évacués de la sortie de la ligne de réaction (24) et pour les empiler les uns sur les autres.

29. Appareil pour l'analyse d'échantillons de sang selon une quelconque des revendications 17 à 18, caractérisé en ce que chaque échantillon de sang est séparé par un centrifuge en un échantillon de globules du sang et en un échantillon de sérum, ledit appareil comprenant en outre une unité de dilution d'échantillon (300) pour la dilution des échantillons de globules du sang et de sérum de manière à former une suspension des globules du sang et une dilution de sérum respectivement, et au minimum deux dispositifs d'alimentation d'échantillons (23-1 à 23-4) pour l'alimentation de la suspension des globules du sang et de la dilution de sérum dans des vases de réaction respectifs (25) dans un même support (26).

30. Appareil selon une quelconque des revendications 17 à 29, caractérisé en ce que chaque support (26) comprend une microplaque (26) et une cassette (27) destinée à contenir amovible la microplaque (26).

31. Appareil selon une quelconque des revendications 17 à 29, caractérisé en ce que chaque support (26) comprend deux moitiés de microplaque (26A, 26B) et une cassette (27) destinée à contenir amovibles les moitiés de microplaque (26A, 26B).

32. Appareil selon la revendication 31, caractérisé en ce que lesdites moitiés de microplaque (26A, 26B) comportent des vases de réaction (25) présentant une configuration et/ou une dimension différentes de surface de fond.

33. Appareil selon une quelconque des revendications 19 à 24, ou selon une quelconque des revendications 25 à 32 prises en liaison avec la revendication 19, caractérisé en ce que lesdits moyens d'avance (28, 29, 30) dans chacun desdits passages (24-1, 24-2, 24-3) de la ligne de réaction comprennent une paire de courroies sans fin (28-1, 28-2, 29-1, etc.) sur lesquelles lesdits supports (26) sont placés.

34. Appareil selon la revendication 33, caractérisé en ce que chaque dispositif de transport (35-1, 36-1) sur lequel chaque support (26) est placé, et un mécanisme (457 à 463) pour mouvoir de haut en bas ledit élément de plateau (35-1, 36-1) entre ladite paire de courroies (28-1, 28-2, 29-1, etc.) et pour moivoir horizontalement l'élément de plateau (35-1, 36-1) entre les passages (24-1, 24-2, 24-3) de la ligne de réaction.

35. Appareil selon une quelconque des revendications 17 à 22, caractérisé en ce que lesdites moyens d'avance (28, 29, 30) comprennent des moyens (21-3, 21-4, 21-5) pour l'indexation des supports (26) par rapport aux moyens d'alimentation d'échantillon et de sérum (23, 31, 34).

36. Appareil selon la revendication 35, caractérisé en ce que chaque moyen d'indexation (21-3, 21-4, 21-5) comprend une paire de pinces (21-12, 21-13) s'engageant sélectivement avec des éléments d'indexation (27-7) formés sur au moins une paroi latérale (27) de chaque support (26).

37. Appareil selon la revendication 36, caractérisé en ce que lesdits éléments d'indexation (27-7) sont constitués par des saillies (27-7).

38. Appareil selon la revendication 36 ou 37 et les revendications 30, 31, ou 32, caractérisé en ce que lesdits éléments d'indexation (27-7) sont formés sur au moins une paroi latérale (27) de chaque cassette (37).

# FIG_1

Serum Delivery    Diluent Delivery

3-2    4-2    5-2

SD

Cell Delivery    Diluent Delivery

Centrifuge

S    2    3-1    4-1    5-1    6

1

BL    R    RD    D

A-Antiserum Delivery

4-1    7-1    8-1    9-1    10-1

8-1    Settle    8-1

RD    A-S    A-T-1    Pattern Judge

A-T-1    Agitation

Sample Cell Delivery    B-Antiserum Delivery

11-1    12-1    13-1

B-T-1    11-1    11-1

B-S    Settle

B-T-1    Agitation    Pattern Judge

A-Cell Delivery

4-2    7-2    8-2    9-2    10-2

8-2    Settle    8-2

SD    A-R    A-T-2    Pattern Judge

A-T-2    Agitation

Sample Serum Delivery    B-Cell Delivery

11-2    12-2    13-2

B-T-2    11-2    11-2

B-R    Settle

B-T-2    Agitation    Pattern Judge

FIG. 2A.

FIG.2B.

FIG. 2C.

4

**FIG. 3**

Set Sample Rack into Cassette → Supply Sample Rack into Sample Line → Formation of Blood Cell & Serum Sample → Delivery of Blood Cell & Serum Sample → Set Sample Rack into Cassette

Set Microplate with Empty Reaction Vessel → Supply Microplate into Reaction Line → Selective Delivery of Reagent → Selective Delivery of Blood Cell & Serum Sample ← Delivery of Blood Cell & Serum Sample

Selective Delivery of Reagent → Transport Microplate from 1st to 2nd Passage

Travel of Microplate Along 2nd Passage

Transport Microplate from 2nd to 3rd Passage → Travel of Microplate Along 3rd Passage → Detection of Pattern → Photographing of Pattern → Discharge Microplate from Reaction Line

Judgement → Display of Judged Result

5

## FIG.4A

## FIG.4B

## FIG.4C

FIG.4D

27

27-2

27-1

27-4

27-3

27-7-1

27-7-2

27-7-3

27-7-4

27-5

27-6

27-7-5

27-7-6

27-7-7

27-7-8

27-7-9

27-7-10

27-7-12

27-7-11

FIG.4E

26'

25

26'-7

26'-1

26'-6

## FIG. 4F

## FIG. 4G

## FIG. 4H

FIG. 5A.

FIG. 5B.

0 051 496

## FIG. 6A

27-3

27

27-6

27-7-3

B

21

27-7-3

21-11

21-12

B

27-6

21-14

27-6

27-7-4

21-13

21-15

21-16

## FIG. 6B

27

X

27-7-3

27-6

21-12

D

21-13

D/2

27-7-4

## FIG. 6C

27

X

27-7-3

27-6

21-12

27-7-4

21-13

27-7-5

## FIG. 6D

27

X

27-7-4

21-12

21-13

27-7-5

27-7-6

## FIG.7A

100
101
102
26    25
27
103
104-1

## FIG.7B

100
101
102
27    25-1-1
25-1-8    26    25-1-2
103
104-1    104-7    104-8

## FIG.7C

104
104B
104A
105

FIG. 8.

# FIG_9

⊞ Uniformly Deposited Pattern (Agglutination)

⊙ Integrated Pattern (Non-Agglutination)

| | | 1 | 2 | 3 | 4 | 5 | 6 | 7 | 8 |
|---|---|---|---|---|---|---|---|---|---|
| A | + | ⊞ | ⊙ | ⊞ | ⊙ | ○ | ○ | ⊙ | ⊞ |
| | − | ⊞ | ⊙ | ⊙ | ⊙ | ○ | ○ | ⊙ | ⊞ |
| B | + | ⊙ | ⊞ | ⊞ | ⊙ | ○ | ○ | ⊞ | ⊙ |
| | − | ⊙ | ⊞ | ⊙ | ⊙ | ○ | ○ | ⊞ | ⊙ |
| O | + | ⊙ | ⊙ | ⊞ | ⊙ | ○ | ○ | ⊞ | ⊞ |
| | − | ⊙ | ⊙ | ⊙ | ⊙ | ○ | ○ | ⊞ | ⊞ |
| AB | + | ⊞ | ⊞ | ⊞ | ⊙ | ○ | ○ | ⊙ | ⊙ |
| | − | ⊞ | ⊞ | ⊙ | ⊙ | ○ | ○ | ⊙ | ⊙ |
| | | ABO (Direct) | | Rh (D) | | Antibody Screening | | ABO (Indirect) | |

0 051 496

## FIG.10A

203
202
201

B

## FIG.10B

205
204

206

*FIG.11A*

*FIG.11B*

0 051 496

FIG. 12.

16

# FIG. 13.

# FIG. 14.

0 051 496

FIG.15

FIG.16

19

# FIG_17

0 051 496

FIG.18A.

4:4

FIG.18B.

5:3

FIG.18C.

6:2

21